(19) Europäisches Patentamt
European Patent Office
Office européen des brevets

(11) Publication number: 0 430 556 A2

# (12) EUROPEAN PATENT APPLICATION

(21) Application number: 90312651.4

(22) Date of filing: 21.11.90

(51) Int. Cl.$^5$: C07D 463/00, A61K 31/435

(30) Priority: 27.11.89 US 441791

(43) Date of publication of application: 05.06.91 Bulletin 91/23

(84) Designated Contracting States: AT BE CH DE DK ES FR GB GR IT LI NL SE

(71) Applicant: ELI LILLY AND COMPANY
Lilly Corporate Center
Indianapolis Indiana 46285 (US)

(72) Inventor: Morin, Johan Michael, Jr.
9 Roselawn Avenue
Brownsburg, Indiana 46112 (US)

(74) Representative: Tapping, Kenneth George et al
Erl Wood Manor
Windlesham Surrey, GU20 6PH (GB)

## (54) (1-Akylsubstituted) and 1,1-dialkylsubstituted) carbacephalosporins.

(57) This invention provides novel (1-alkylsubstituted) and (1,1-disubstituted)carbacephalosporins useful as antimicrobial agents.

EP 0 430 556 A2

Jouve, 18, rue Saint-Denis, 75001 PARIS

# (1-ALKYLSUBSTITUTED) AND (1,1-DIALKYLSUBSTITUTED) CARBACEPHALOSPORINS

This invention relates to 1-carba(dethia)-cephalosporin antibiotics, intermediates for the preparation thereof, to pharmaceutical formulations comprising the antibiotics, and to a method for the treatment of infectious diseases in man and animals.

The preparation of 1-carbacephalosporins and C-3 substituted methyl derivatives thereof is taught broadly by Christensen et al., in U.S. Patent No. 4,226,866. Hirata et al., in U.K. patent application No. 2041923, teach a method for preparing 3-H and 3-halo 1-carbacephalosporins, while Hatanaka et al., Tetrahedron Letters, 24, 4837-4838 (1983), teach a method for preparing a 3-hydroxy-(±)-1-carbacephalosporin. A variety of 3-hydroxy-1-carbacephalosporins are also provided in EPO Patent Application Publication 209,352 while their 3-triflate (3-trifluoromethanesulfonic acid) esters are disclosed in EPO Patent Application Publication 211,540.

Uyeo et al. in Chem. Pharm. Bull. 28, 1578-1583 (1980) disclose 1-phenylthio-1-carbacephalosporins while Bremner et al. in Tetrahedron Letters, 24, 3783-3786 (1983), disclose 1-hydroxy-1-carbacephalosporins. Herdewijn et al. in J. Med. Chem., 29, 661-664 (1986) disclose 1-methylene-1-carbacephalosporins. Monosubstituted and disubstituted carbapenems are also known. Shih et al. in Tetrahedron Letters, 26, 587-590 (1985) disclose 1-methylthienamycin derivatives while Shibuya et al. in Tetrahedron Letters, 22, 3611-3614 (1981) and Shibuya et al. in Tetrahedron Letters, 21, 4009-4012 (1980) both disclose 1,1-dimethylcarbapenems.

This invention provides (1-alkyl substituted) and (1,1-dialkylsubstituted) carbacephalosporins. Although many safe and potent antibiotics of the β-lactam class are known and used clinically, the research into this class of compounds continues in an effort to find antibiotics with improved efficacy, particularly against microorganisms insensitive or resistant to the known antibiotics.

This invention provides novel (1-alkylsubstituted) and (1,1-dialkylsubstituted) carbacephalosporins. More specifically, this invention provides a compound of the Formula (I)

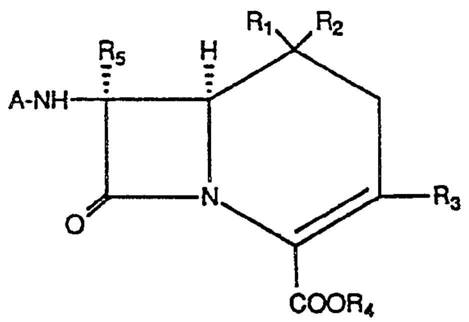

I

wherein A is hydrogen, an amino-protecting group, or an acyl group

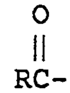

wherein R is hydrogen, $C_1$-$C_6$ alkyl, $C_1$-$C_6$ alkyl substituted by cyano, carboxy, halogen, amino, $C_1$-$C_4$ alkoxy, $C_1$-$C_4$ alkylthio, trifluoromethyl, or trifluoromethylthio, naphthyl, an optionally substituted phenyl group represented by the formula

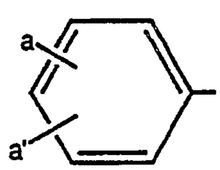

wherein a and a' independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, $C_1$-$C_4$ alkanoylamino, $C_1$-$C_4$ alkylsulfonylamino, carboxy, carbamoyl, hydroxymethyl, aminomethyl, or carboxymethyl ;
a group represented by the formula

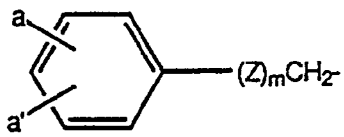

wherein Z is 0 or S, and m is 0 or 1 ;
a heteroarylmethyl group represented by the formula

$R_6$-$CH_2$-

wherein $R_6$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, or $C_1$-$C_4$ alkylsulfonylamino ;
a substituted methyl group represented by the formula

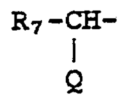

wherein $R_7$ is cyclohex-1,4-dienyl, or an optionally substituted phenyl group represented by the formula

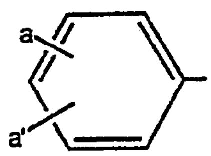

wherein a and a′ have the above defined meanings, or $R_7$ is $R_6$ as defined above, and Q is hydroxy, $C_1$-$C_4$ alkanoyloxy, carboxy, sulfo, or amino ;
or R is a keto group or an oximino-substituted group represented by the formulae

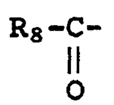

or

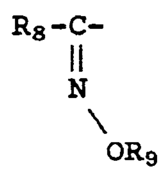

wherein $R_8$ is $R_6$ or $R_7$ as defined above and $R_9$ is hydrogen, $C_1$-$C_4$ alkyl, or a carboxysubstituted alkyl or cycloalkyl group represented by the formula

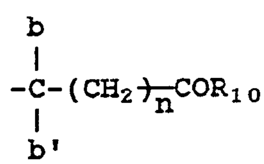

wherein b and b′ independently are hydrogen or $C_1$-$C_3$ alkyl, or b and b′, when taken together with the carbon to which they are bonded, form a 3- to 6-membered carbocyclic ring, n is 0-3, and $R_{10}$ is hydroxy, $C_1$-$C_4$ alkoxy, amino, $C_1$-$C_4$ alkylamino, or di($C_1$-$C_4$ alkyl)amino ;
$R_1$ and $R_2$ independently are hydrogen or $C_1$-$C_4$ unbranched alkyl, but $R_1$ and $R_2$ are not both hydrogen, or $R_1$ and $R_2$, together, form cyclopropyl ;
$R_3$ is hydrogen, halo, $C_1$-$C_6$ alkyl, $C_1$-$C_6$ alkyl substituted by cyano, carboxy, halogen, or amino, phenyl, substituted phenyl, cyano ; a group of the formula

$$\begin{array}{c}(O)_z\\ \|\\ -SR_{11}\end{array}$$

wherein z is 0, 1 or 2 and $R_{11}$ is $C_1$-$C_6$ alkyl, $C_1$-$C_6$ alkyl substituted by cyano, carboxy, halogen, or amino, phenyl, or substituted phenyl ; a group of the formula

$$\begin{array}{c}(O)_2\\ \|\\ -OS-R_{12}\end{array}$$

wherein $R_{12}$ is methyl or trifluoromethyl ; a group of the formula

$$-COOR_{13}$$

wherein $R_{13}$ is $C_1$-$C_6$ alkyl or $C_7$-$C_{12}$ arylalkyl ; a group of the formula

$$-OR_{14}$$

wherein $R_{14}$ is hydrogen, $C_1$-$C_6$ alkyl, or $C_7$-$C_{12}$ arylalkyl ;
$R_4$ is hydrogen, a biologically labile group, or a carboxy-protecting group ;
$R_5$ is hydrogen, $C_1$-$C_4$ alkoxy, $C_1$-$C_4$ alkylthio, or a formamido group ;
or a pharmaceutically acceptable salt thereof.

The compounds represented by the formula I wherein A is an acyl group, RCO, and $R_4$ is hydrogen or a biologically labile group, and the pharmaceutically acceptable salts thereof, inhibit the growth of microorganisms pathogenic to man and animals. The compounds which are protected (A = an amino protecting group), deprotected (A = hydrogen), or are in protected form ($R_4$ = carboxy-protecting group) are useful as intermediates as described hereinafter.

The invention also provides pharmaceutical formulations comprising a biologically active compound of formula I as described above, associated with one or more pharmaceutically acceptable carriers, excipients or diluents therefor, and a process of preparing such formulations.

Also provided is the use of such biologically active compounds as pharmaceuticals, and a process for preparing a biologically active compound of formula I by removing a carboxy-protecting $R_4$ group and, optionally, adding a biologically labile $R_4$ group or salifying the product wherein $R_4$ is hydrogen.

In the above definition of the compounds represented by the formula I, "$C_1$-$C_6$ alkyl" refers to the straight and branched chain alkyl groups such as methyl, ethyl, n-propyl, isopropyl, n-butyl, t-butyl, n-pentyl, n-hexyl, 3-methylpentyl, and like alkyl groups ; "$C_1$-$C_6$ alkyl substituted by cyano" refers to cyanomethyl, cyanoethyl, 4-cyanobutyl, and the like ; "$C_1$-$C_6$ alkyl substituted by... carboxy" refers to such groups as carboxymethyl, 2-carboxyethyl, 2-carboxypropyl, 4-carboxybutyl, 5-carboxypentyl, and the like ; "$C_1$-$C_6$ alkyl substituted by... halogen" refers to chloromethyl, bromomethyl, 2-chloroethyl, 1-bromoethyl, 4-chlorobutyl, 4-bromopentyl, 6-chlorohexyl, 4-fluorobutyl, 3-fluoropropyl, fluoromethyl, and the like ; "$C_1$-$C_6$ alkyl substituted by... amino" refers to such groups as 2-aminoethyl, aminomethyl, 3-aminopropyl and 4-aminobutyl ; "$C_1$-$C_6$ alkyl substituted by... $C_1$-$C_4$ alkoxy" refers to methoxymethyl, 2-methoxyethyl, 2-ethoxyethyl, ethoxymethyl, 3-propoxypropyl, 3-ethoxybutyl, 4-t-butoxybutyl, 3-methoxypentyl, 6-methoxyhexyl, and like groups ; "$C_1$-$C_6$ alkyl substituted by... $C_1$-$C_4$-alkylthio" refers to such groups as for example methylthiomethyl, 2-methylthioethyl, 2-ethylthiopropyl., 4-methylthiobutyl, 5-ethylthiohexyl, 3-t-butylthiopropyl, and like groups ; "$C_1$-$C_6$ alkyl substituted by... trifluoromethyl" is exemplified by 2,2,2-trifluoroethyl, 3,3,3-trifluoropropyl, 4,4,4-trifluorobutyl, 6,6,6-trifluorohexyl, and the like ; and "$C_1$-$C_6$ alkyl substituted by... trifluoromethylthio" refers to, for example, trifluoromethylthiomethyl, 2-trifluoromethylthioethyl, 2-trifluoromethylthiopropyl, 4-trifluoromethylthiobutyl, 5-trifluoromethylthiohexyl, and like $C_1$-$C_6$ alkyl substituted groups.

The terms "halo" and "halogen" refer to the fluoro, chloro, bromo or iodo groups.

When, in the formula I, R is a substituted phenyl group wherein the substituent(s) are represented by a and a', examples of such groups are halophenyl such as 4-chlorophenyl, 3-bromophenyl, 2-fluorophenyl, 2-iodophenyl, 2,4-dichlorophenyl, and 3,5-dichlorophenyl ; hydroxyphenyl such as 2-hydroxyphenyl, 3-hydroxyphenyl, 4-hydroxyphenyl, 2,4-dihydroxyphenyl, and 3,4-dihydroxyphenyl ; alkoxyphenyl, such as 2,6-dimethoxyphenyl, 4-methoxyphenyl, 3-ethoxyphenyl, 3,4-dimethoxyphenyl, 4-t-butoxyphenyl, 4-methoxy-3-ethoxyphenyl, and 4-n-propoxyphenyl ; alkanoyloxyphenyl such as 2-acetoxyphenyl, 4-propionoxyphenyl, 4-formyloxyphenyl, 4-acetoxyphenyl, 3-butyryloxyphenyl, and 3-acetoxyphenyl ; alkylphenyl such as 4-methylphenyl, 2-methylphenyl, 2,4-dimethylphenyl, 3-t-butylphenyl, 4-ethylphenyl, 4-ethyl-3-methylphenyl, and 3,5-dimethylphenyl ; alkylthiophenyl such as 4-methylthiophenyl, 3-n-butylthiophenyl, 2-ethylthiophenyl,

3,4-dimethylthiophenyl, and 3-n-propylthiophenyl ; aminophenyl such as 2-aminophenyl, 4-aminophenyl, 3,5-diaminophenyl, and 3-aminophenyl ; alkanoylaminophenyl such as 2-acetylaminophenyl, 4-acetylaminophenyl, 3-propionylaminophenyl, and 4-butyrylaminophenyl ; alkylsulfonylaminophenyl such as 3-methylsulfonylaminophenyl, 4-methylsulfonylaminophenyl, 3,5-di-(methylsulfonylamino)phenyl, 4-n-butylsulfonylaminophenyl, and 3-ethylsulfonylaminophenyl ; carboxyphenyl such as 2-, 3-, or 4-carboxyphenyl, 3,4-dicarboxyphenyl, and 2,4-dicarboxyphenyl ; carbamoylphenyl such as 2-carbamoylphenyl, 2,4-dicarbamoylphenyl, and 4-carbamoylphenyl ; hydroxymethylphenyl such as 4-hydroxymethylphenyl and 2-hydroxymethylphenyl ; aminomethylphenyl such as 2-aminomethylphenyl and 3-aminomethylphenyl ; and carboxymethylphenyl such as 2-carboxymethylphenyl, 4-carboxymethylphenyl, and 3,4-di(carboxymethyl)phenyl ; and the substituted phenyl groups bearing different substituents such as 4-chloro-3-methylphenyl, 4-fluoro-3-hydroxyphenyl, 3,5-dichloro-4-hydroxyphenyl, 4-hydroxy-3-chlorophenyl, 4-hydroxy-3-methylphenyl, 4-ethyl-3-hydroxyphenyl, 4-methoxy-3-hydroxyphenyl, 4-t-butyloxy-2-hydroxyphenyl, 4-acetylamino-3-methoxyphenyl, 3-amino-4-ethylphenyl, 2-aminomethyl-4-chlorophenyl, 2-hydroxymethyl-3-methoxyphenyl, 2-hydroxymethyl-4-fluorophenyl, 2-acetoxy-4-aminophenyl, 4-acetoxy-3-methoxyphenyl, 3-isopropylthio-4-chlorophenyl, 2-methylthio-4-hydroxymethylphenyl, 4-carboxy-3-hydroxyphenyl, 4-ethoxy-3-hydroxyphenyl, 4-methylsulfonylamino-2-carboxyphenyl, 4-amino-3-chlorophenyl, and 2-carboxymethyl-4-hydroxyphenyl.

Examples of RCO- groups of the formula I wherein R is a group represented by the formula

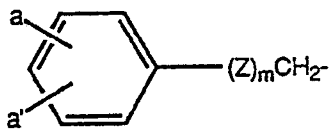

with m = 0 are : phenylacetyl, 4-hydroxyphenylacetyl, 4-chlorophenylacetyl, 3,4-dichlorophenylacetyl, 4-methoxyphenylacetyl, 3-ethoxyphenylacetyl, 2-aminomethylphenylacetyl, 3-carboxyphenylacetyl, 4-acetoxyphenylacetyl, 3-aminophenylacetyl, and 4-acetylaminophenylacetyl ; and with m = 1 and Z = O, phenoxyacetyl, 4-chlorophenoxyacetyl, 4-fluorophenoxyacetyl, 3-aminophenoxyacetyl, 3-hydroxyphenoxyacetyl, 2-methoxyphenoxyacetyl, 2-methylthiophenoxyacetyl, 4-acetylaminophenoxyacetyl, 3,4-dimethylphenoxyacetyl, and 3-hydroxymethylphenoxyacetyl ; and with m = 1 and Z = S, phenylthioacetyl, 4-chlorophenylthioacetyl, 3,4-dichlorophenylthioacetyl, 2-fluorophenylthioacetyl, 3-hydroxyphenylthioacetyl, and 4-ethoxyphenylthioacetyl.

Examples of $R_6$-$CH_2$CO- groups of the formula I wherein $R_6$ is a heteroaryl group are : 2-thienylacetyl, 3-thienylacetyl, 2-furylacetyl, 2-benzothienylacetyl, 2-benzofurylacetyl, indol-2-ylacetyl, 1H-tetrazol-1-ylacetyl, oxazol-2-ylacetyl, oxazol-4-ylacetyl, thiazol-4-ylacetyl, 2-aminothiazol-4-ylacetyl, 1,3,4-oxadiazol-2-ylacetyl, 1,3,4-thiadiazol-2-ylacetyl, 5-ethyl-1,3,4-thiadiazol-2-ylacetyl, and like heteroaryl groups substituted by amino, $C_1$-$C_4$ alkylsulfonylamino, hydroxy, halo, $C_1$-$C_4$ alkyl or $C_1$-$C_4$ alkoxy groups.

Examples of RCO- groups of the formula I compounds wherein R is a substituted methyl group represented by the formula $R_7$-CH(Q)- and Q is amino, carboxy, hydroxy, or sulfo, are 2-carboxy-2-phenylacetyl, 2-amino-2-(2-naphthalenyl)acetyl, 2-carboxy-2-(4-hydroxyphenyl)acetyl, 2-amino-2-phenylacetyl, 2-amino-2-(4-hydroxyphenyl)acetyl, 2-amino-2-(3-chloro-4-hydroxyphenyl)acetyl, 2-amino-2-(cyclohex-1,4-dien-1-yl)acetyl, 2-amino-2-(3-methylsulfonamidophenyl)-acetyl, 2-amino-2-(3-ethylsulfonaminophenyl)acetyl, 2-hydroxy-2-phenylacetyl, 2-formyloxy-2-phenylacetyl, 2-sulfo-2-phenylacetyl, 2-sulfo-2-(4-methylphenyl)-acetyl, and 2-acetoxy-2-(3-hydroxyphenyl)acetyl, 2-amino-2-(2-thienyl)acetyl, 2-amino-2-(3-benzothienyl) acetyl, 2-amino-2-(1H-tetrazol-1-yl)acetyl, 2-hydroxy-2-(1,3,4-thiadiazol-2-yl)acetyl, 2-amino-2-(2-aminothiazol-4-yl)acetyl, 2-carboxy-2-(2-thienyl)acetyl, 2-carboxy-2-(benzothien-2-yl)acetyl, and 2-hydroxy-2-(benzofur-2-yl)acetyl.

Examples of RCO acyl groups of the compounds represented by formula I when R is a keto group or an oximino-substituted group represented by the formulae

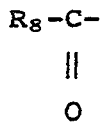

or

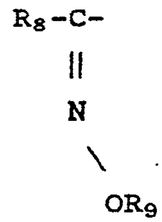

are the keto groups 2-oxo-2-phenylacetyl, 2-oxo-2-(2-thienyl)acetyl, 2-oxo-2-(2-aminothiazol-4-yl)acetyl ; and oximino-substituted groups 2-phenyl-2-methoxyaminoacetyl, 2-(2-thienyl)-2-ethoxyiminoacetyl, 2-(2-furyl)-2-methoxyiminoacetyl, 2-(2-benzothienyl)-2-carboxymethoxyiminoacetyl, 2-(2-thienyl)-2-(2-carboxyethoxy)iminoacetyl, 2-(2-amino-1,2,4-thiadiazol-4-yl)-2-methoxyiminoacetyl, 2-(2-aminothiazol-4-yl)-2-methoxyiminoacetyl, 2-(2-chlorothiazol-4-yl)-2-methoxyiminoacetyl, 2-(2-aminothiazol-4-yl)-2-(2-carboxyprop-2-yl)oxyiminoacetyl, 2-(2-aminothiazol-4-yl)-2-(2-carbamoylprop-2-yl) oxyiminoacetyl, and 2-(5-amino-1,3,4-thiadiazol-2-yl)-2-methoxyiminoacetyl.

Examples of $C_1$-$C_4$ unbranched alkyl are methyl, ethyl, n-propyl and n-butyl.

The term "$C_7$-$C_{12}$ arylalkyl" denotes a $C_1$-$C_6$ alkyl group substituted at any position by a phenyl ring. Examples of such a group include phenylmethyl (benzyl), 2-phenylethyl, 3-phenyl-(n-propyl), 4-phenylhexyl, 3-phenyl-(n-amyl), 3-phenyl-(sec-butyl) and the like. A preferred group is the benzyl group.

The term "$C_7$-$C_{12}$ substituted arylalkyl" denotes a $C_7$ to $C_{12}$ substituted alkylaryl group substituted on the $C_1$-$C_6$ alkyl portion with one or two groups chosen from halogen, hydroxy, protected hydroxy, amino, protected amino, $C_1$-$C_7$ acyloxy, nitro, carboxy, protected carboxy, carbamoyl, carbamoyloxy, cyano, methylsulfonylamino or $C_1$-$C_4$ alkoxy ; and/or the phenyl group may be substituted with 1 or 2 groups chosen from halogen, hydroxy, protected hydroxy, nitro, $C_1$-$C_6$ alkyl, $C_1$-$C_4$ alkoxy, carboxy, protected carboxy, carboxymethyl, protected carboxymethyl, hydroxymethyl, protected hydroxymethyl, aminomethyl, protected aminomethyl, or a methylsulfonylamino group. As before, when either the $C_1$-$C_6$ alkyl portion or the phenyl portion or both are disubstituted, the substituents can be the same or different.

Examples of the term "$C_7$-$C_{12}$ substituted alkylaryl" include groups such as 2-phenyl-1-chloroethyl, 2-(4-methoxyphenyl)ethyl, 2,6-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6-(4-methoxyphenyl)-3-carboxy(n-hexyl), 5-(4-aminomethylphenyl)-3-(aminomethyl)(n-pentyl), and the like.

Examples of the term "perfluoro $C_2$-$C_4$ alkyl" include perfluoroethyl, perfluoro-n-propyl, perfluoro-iso-propyl, perfluoro-n-butyl, perfluoro-sec-butyl and the like.

The term "organic or inorganic cation" refers to counter-ions for the carboxylate anion of a carboxylate salt. The counter-ions are chosen from the alkali and alkaline earth metals, such as lithium, sodium, potassium, barium and calcium ; ammonium ; and organic cations such as dibenzylammonium, benzylammonium, 2-hydroxyethylammonium, bis(2-hydroxyethyl) ammonium, phenylethylbenzylammonium, dibenzylethylenediammonium, and like cations. Other cations encompassed by the above term include the protonated forms of procaine, quinine and N-methylglucosamine, and the protonated forms of basic amino acids such as glycine, ornithine, histidine, phenylglycine, lysine and arginine. A preferred cation for the carboxylate anion is the sodium cation.

The term "carboxy-protecting group" as used in this document refers to conventional groups commonly used in the β-lactam art to block or protect the carboxylic acid group while reactions are carried out on other functional groups on the compound. Examples of such carboxylic acid protecting groups include benzyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, 2-(trimethylsilyl)ethyl, 2-(di(n-butyl)methylsilyl)ethyl, 2-(p-toluenesulfonyl)ethyl, 2-(4-nitrobenzylsulfonyl)ethyl, allyl, cinnamyl, 1-(trimethylsilylmethyl)prop-1-en-3-yl, and like moieties. The species of carboxy-protecting group employed is not critical so long as the derivatized carboxylic acid is stable to the conditions of subsequent reaction(s) on other positions of the molecule and can be removed at the appropriate point without disrupting the remainder of the molecule. In particular, it is important not to subject the carboxy-protected 1-carbacephalosporin molecule to strong nucleophilic bases. Such harsh removal conditions are also to be avoided when removing amino-protecting groups and hydroxy-protecting groups, discussed below. Preferred carboxylic acid protecting groups are the benzhydryl, allyl and p-nitrobenzyl groups. Carboxy-protecting groups similar to those used in the cephalosporin, penicillin and peptide arts can also be used to protect a carboxy group substituents of the compounds

provided herein. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5.

The term "amino-protecting group" as used in the specification refers to substituents of the amino group commonly employed to block or protect the amino functionality while reacting other functional groups on the compound. Examples of such amino-protecting groups include the formyl group, the trityl group, the phthalimido group, the trichloroacetyl group, the chloroacetyl, bromoacetyl and iodoacetyl groups, urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 1,1-diphenyleth-1-yloxycarbonyl, 1,1-diphenylprop-1-yloxycarbonyl, 2-phenylprop-2-yloxycarbonyl, 2-(p-toluyl)prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)ethoxycarbonyl, 2-(methylsulfonyl)ethoxycarbonyl, 2-(triphenylphosphino)ethoxycarbonyl, 9-fluorenylmethoxycarbonyl ("FMOC"), 2-(trimethylsilyl)ethoxycarbonyl, allyloxycarbonyl, 1-(trimethylsilylmethyl)prop-1-en-3-yloxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, 1-piperidyloxycarbonyl and the like ; the benzoylmethylsulfonyl group, the 2-(nitro)phenylsulfonyl group, the diphenylphosphine oxide group and like amino-protecting groups. The species of amino-protecting group employed is not critical so long as the derivatized amino group is stable to the condition of subsequent reaction(s) on other positions of the molecule and can be removed at the appropriate point without disrupting the remainder of the molecule. Preferred amino-protecting groups are the 1,2-bis-(dimethylsilyl)ethylene (See, e.g., U.S. Patent No. 4,558,124), benzyloxycarbonyl, p-nitrobenzyloxycarbonyl, allyloxycarbonyl, t-butoxycarbonyl, and trityl groups. Similar amino-protecting groups used in the cephalosporin, penicillin and peptide art are also embraced by the above terms. Further examples of groups referred to by the above terms are described by J.W. Barton, "Protective Groups In Organic Chemistry", J.G.W. McOmie, Ed., Plenum Press, New York, N.Y., 1973, Chapter 2, and T.W. Greene, "Protective Groups in Organic Synthesis", John Wiley and Sons, New York, N.Y., 1981, Chapter 7.

The term "pharmaceutically acceptable salt" refers to salts of the carboxy group or other acidic moiety in the molecule, such as a carboxy or sulfo substituent group, and includes salts formed with organic amines and inorganic bases. Such amines and bases include those whose counter-ions are chosen from the alkali and alkaline earth metals (such as lithium, sodium, potassium, barium and calcium) ; ammonium ; and the organic cations (such as dibenzylammonium, benzylammonium, 2-hydroxyethylammonium, bis(2-hydroxyethyl)-ammonium, phenylethylbenzylammonium, dibenzylethylenediammonium, and like cations). A preferred cation for the carboxylate anion is the sodium cation.

Furthermore, the term includes salts that form by standard acid-base reactions with basic groups of the compounds of this invention (such as amino groups) and organic or inorganic acids. Such acids include hydrochloric, sulfuric, phosphoric, acetic, succinic, citric, lactic, maleic, fumaric, palmitic, cholic, mucic, D-glutamic, d-camphoric, glutaric, phthalic, tartaric, lauric, stearic, salicyclic, methanesulfonic, benzenesulfonic, sorbic, picric, benzoic, cinnamic, and like acids.

The (1-alkyl substituted) and (1,1-dialkylsubstituted)carbacephalosporins provided herein can be esterified with a biologically labile group to form esters which form the free acid antibiotic form in vivo. Biologically labile groups are acyloxymethyl groups represented by the formula

$$-CH_2-O-\overset{\overset{\displaystyle O}{\|}}{C}-(C_1-C_4alk) \quad ;$$

acyloxyalkyl groups represented by the formula

$$(C_1-C_4alk)-\overset{\overset{\displaystyle O}{\|}}{C}-O-\underset{\underset{\displaystyle (C_1-C_4alk)}{|}}{CH}- \quad ;$$

dialkyl ether groups represented by the formula

$(C_1-C_4\ alk)-O-CH_2CH_2-O-CH_2-$;

phthalidyl, indanyl, or the 5-methyl-2-oxo-1,3-dioxolen-4-methyl-4'-ylcyclocarbonate group represented by the formula

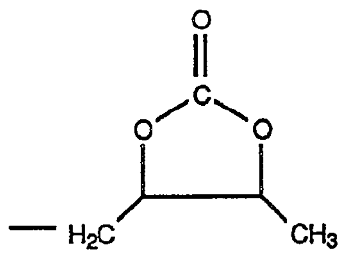

Examples of acyloxymethyl groups, $R_4$, are acetoxymethyl, propionoxymethyl and pivaloyloxymethyl. Acyloxyalkyl groups are exemplified by 1-acetoxyethyl, 1-acetoxypropyl, and 1-propionoxybutyl. Examples of dialkyl ether groups are β-methoxyethoxymethyl, β-ethoxyethoxymethyl, and β-t-butyloxyethoxymethyl.

The biologically labile esters of the (1-alkyl substituted) and (1,1-dialkylsubstituted)carbacephalosporins can be used as pro-drugs and can provide ease of formulation and administration of the antibiotic.

Examples of the above defined (1-alkyl substituted) and (1,1-dialkylsubstituted) carbacephalosporins are described below in Table 1 wherein the terms in the column headings refer to Formula (I).

Table 1

| A | $R_1$ | $R_2$ | $R_3$ | $R_4$ | $R_5$ |
|---|---|---|---|---|---|
| acetyl | Hydrogen | Methyl | Hydrogen | H | Hydrogen |
| n-propionyl | methyl | methyl | Chlorine | H | methoxy |
| t-butyryl | ethyl | ethyl | Fluorine | H | ethoxy |
| n-valeryl | n-propyl | n-propyl | Bromine | H | propoxy |
| 3-methylvaleryl | n-butyl | n-butyl | Iodine | H | butoxy |
| cyanoacetyl | methyl | Hydrogen | methyl | H | methylthio |
| 4-cyanobutyryl | methyl | methyl | ethyl | H | ethylthio |
| 2-carboxyacetyl | methyl | ethyl | propyl | H | isopropylthio |
| 4-carboxybutyryl | methyl | n-propyl | butyl | H | n-butylthio |
| chloroacetyl | methyl | n-butyl | pentyl | H | isobutylthio |
| bromoacetyl | Hydrogen | methyl | hexyl | H | formamido |
| 4-flurobutyryl | methyl | methyl | isobutyl | H | Hydrogen |
| 6-chlorohexanoyl | ethyl | methyl | isopropyl | H | methoxy |
| 2-aminoacetyl | n-propyl | methyl | 3-methylpentyl | H | ethoxy |
| 4-aminobutyryl | n-butyl | methyl | cyanomethyl | H | propoxy |
| methoxyacetyl | Hydrogen | methyl | 4-cyanobutyl | H | butoxy |
| ethoxyacetyl | Hydrogen | ethyl | cyanomethyl | H | methylthio |

Table 1 (Continued)

| A | $R_1$ | $R_2$ | $R_3$ | $R_4$ | $R_5$ |
|---|---|---|---|---|---|
| 6-methoxyhexanoyl | Hydrogen | n-propyl | 4-cyanobutyl | H | ethylthio |
| methylthioacetyl | Hydrogen | n-butyl | 2-carboxyethyl | H | isopropylthio |
| 2-ethylthiopropionyl | methyl | ethyl | 2-carboxypropyl | H | n-butylthio |
| 3-t-butylthiopropionyl | n-propyl | n-butyl | 5-carboxypentyl | H | isobutylthio |
| 2,2,2-trifluoroacetyl | Hydrogen | methyl | chloromethyl | H | formamido |
| 4,4,4-trifluorobutyryl | methyl | methyl | 2-bromoethyl | H | Hydrogen |
| trifluoromethylthioacetyl | ethyl | ethyl | 4-fluorobutyl | H | methoxy |
| 4-trifluoromethylthiobutyryl | n-propyl | n-butyl | 5-chloropentyl | H | ethoxy |
| 4-chlorophenylacetyl | n-butyl | n-propyl | 6-bromohexyl | H | propoxy |
| 3-bromophenylacetyl | Hydrogen | ethyl | 2-aminoethyl | H | butoxy |
| 2,4-dichlorophenylacetyl | cyclopropyl | | 4-aminobutyl | H | methylthio |
| 2-hydroxyphenylacetyl | ethyl | methyl | 4-chlorophenyl | H | ethylthio |
| 4-hydroxyphenylacetyl | methyl | ethyl | 2-fluorophenyl | H | isopropylthio |
| 3,4-dihydroxyphenylacetyl | methyl | methyl | 2,4-dibromophenyl | H | n-butylthio |
| 2,6-dimethoxyphenylacetyl | methyl | n-propyl | 2-hydroxyphenyl | H | isobutylthio |
| 4-methoxy-3-ethoxyphenylacetyl | ethyl | methyl | 2,3-hydroxyphenyl | H | n-butylthio |
| 2-acetoxyphenylacetyl | ethyl | methyl | 3-ethoxyphenyl | H | isobutylthio |
| 4-formyloxyphenylacetyl | n-propyl | n-butyl | 2,6-dimethoxyphenyl | H | formamido |

Table 1 (Continued)

| A | $R_1$ | $R_2$ | $R_3$ | $R_4$ | $R_5$ |
|---|---|---|---|---|---|
| 3-butyryloxyphenylacetyl | cyclopropyl | | 4-t-butoxyphenyl | H | Hydrogen |
| 4-methylphenylacetyl | n-butyl | n-propyl | 4-methoxy-3-ethoxyphenyl | H | methoxy |
| 3-t-butylphenylacetyl | ethyl | methyl | 2-acetoxyphenyl | H | ethoxy |
| 4-ethyl-3-methylphenylacetyl | ethyl | Hydrogen | 4-propionoxyphenyl | H | propoxy |
| 4-methylthiophenylacetyl | methyl | Hydrogen | 3-formyloxyphenyl | H | butoxy |
| 3-n-butylthiophenylacetyl | methyl | ethyl | 2-methylphenyl | H | methylthio |
| 3,4-dimethylthiophenylacetyl | Hydrogen | methyl | 2-methylphenyl | H | ethylthio |
| 2-aminophenylacetyl | Hydrogen | methyl | 2,4-diethylphenyl | H | isopropylthio |
| 3,5-diaminophenylacetyl | methyl | ethyl | 3-n-butylthiophenyl | H | n-butylthio |
| 2-acetylaminophenylacetyl | ethyl | methyl | 3,4-dimethylthiophenyl | H | isobutylthio |
| 3-propionylaminophenylacetyl | cyclopropyl | | 4-aminophenyl | H | formamido |
| 3-methylsulfonylaminophenylacetyl | n-butyl | n-propyl | 3,5-diaminophenyl | H | Hydrogen |
| 3,5-di(methylsulfonylamino)phenyl-acetyl | methyl | ethyl | 2-acetylaminophenyl | H | methoxy |
| 3,4-dicarboxyphenylacetyl | n-propyl | n-butyl | 3-methylsulfonylaminophenyl | H | ethoxy |
| 4-carboxyphenylacetyl | Hydrogen | ethyl | 3,5-di(methylsulfonylamino)phenyl | H | propoxy |
| 2-carbamoylphenylacetyl | Hydrogen | methyl | 4-carboxyphenyl | H | butoxy |
| 2,4-dicarbamoylphenylacetyl | Hydrogen | n-butyl | 2,4-dicarbamoylphenyl | H | methylthio |

Table 1 (Continued)

| A | $R_1$ | $R_2$ | $R_3$ | $R_4$ | $R_5$ |
|---|---|---|---|---|---|
| 4-hydroxymethylphenylacetyl | Hydrogen | n-propyl | 3-hydroxymethylphenyl | H | ethylthio |
| 2-aminomethylphenylacetyl | methyl | methyl | 4-aminomethylphenyl | H | isopropylthio |
| 2-carboxymethylphenylacetyl | methyl | ethyl | 3-carboxymethylphenyl | H | n-butylthio |
| 3,4-di(carboxymethyl)phenylacetyl | methyl | n-butyl | 4-chloro-3-methylphenyl | H | isobutylthio |
| 4-chloro-3-methylphenylacetyl | methyl | n-propyl | 4-ethyl-2-hydroxyphenyl | H | formamido |
| 4-hydroxy-3-chlorophenylacetyl | methyl | Hydrogen | 4-acetylamino-3-methoxyphenyl | H | Hydrogen |
| 3-ethyl-4-hydroxyphenylacetyl | cyclopropyl | | 3-isopropylthio-4-chlorophenyl | H | methoxy |
| 4-t-butyloxy-2-hydroxyphenylacetyl | n-propyl | n-butyl | 4-amino-3-chlorophenyl | H | ethoxy |
| 3-amino-2-ethylphenylacetyl | ethyl | ethyl | cyano | H | propoxy |
| 2-hydroxymethyl-4-flurophenylacetyl | ethyl | methyl | methylthio | H | butoxy |
| 2-acetoxy-4-amino-phenylacetyl | ethyl | n-propyl | ethylsulfonyl | H | methylthio |
| 3-isopropylthio-4-chlorophenyl-acetyl | ethyl | n-butyl | n-propylsulfonyl | H | ethylthio |
| phenylacetyl | cyclopropyl | | n-butylthio | H | isopropylthio |
| 3-hydroxyphenylacetyl | Hydrogen | methyl | n-pentylsulfinyl | H | n-butylthio |
| 3,4-dichlorophenylacetyl | methyl | Hydrogen | n-hexylsulfonyl | H | isobutylthio |
| 4-methoxyphenylacetyl | methyl | n-butyl | cyanomethylthio | H | formamido |
| 3-aminomethylphenylacetyl | methyl | n-propyl | 3-cyanopropylsulfinyl | H | Hydrogen |

Table 1 (Continued)

| A | $R_1$ | $R_2$ | $R_3$ | $R_4$ | $R_5$ |
|---|---|---|---|---|---|
| 4-acetoxyphenylacetyl | ethyl | n-butyl | 5-cyanopentylsulfonyl | H | methoxy |
| 4-acetylaminophenylacetyl | cyclopropyl | | 2-carboxyethylthio | H | ethoxy |
| phenoxyacetyl | n-propyl | n-butyl | 4-carboxybutylsulfinyl | H | propoxy |
| 4-fluorophenoxyacetyl | n-propyl | Hydrogen | 6-carboxyhexylsulfonyl | H | butoxy |
| 3-aminophenoxyacetyl | n-propyl | methyl | chloromethylthio | H | methylthio |
| 2-methylthiophenoxyacetyl | ethyl | methyl | 2-bromoethylsulfinyl | H | ethylthio |
| phenylthioacetyl | methyl | ethyl | 4-fluorobutylsulfonyl | H | isopropylthio |
| 2-fluorophenylthioacetyl | methyl | n-propyl | 2-aminoethylthio | H | n-butylthio |
| 2-thienylacetyl | methyl | n-butyl | 3-aminopropylsulfinyl | H | isobutylthio |
| 2-furylacetyl | cyclopropyl | | 5-aminopentylsulfonyl | H | formamido |
| 2-benzothienylacetyl | ethyl | ethyl | 2-hydroxyphenylthio | H | Hydrogen |
| indol-2-ylacetyl | methyl | n-propyl | 2-acetoxyphenylsulfinyl | H | methoxy |
| 1H-tetrazol-1-ylacetyl | Hydrogen | methyl | 4-propionoxyphenylsulfonyl | H | ethoxy |
| oxazol-4-ylacetyl | methyl | ethyl | 3-methylphenylthio | H | propoxy |
| thiazol-4-ylacetyl | Hydrogen | ethyl | 2,3-diethylphenylsulfinyl | H | butoxy |
| 5-ethyl-1,3,4-thiadiazol-2-ylacetyl | n-propyl | n-butyl | 4-aminophenylsulfonyl | H | methylthio |
| 2-carboxy-2-phenylacetyl | ethyl | methyl | 3-carboxyphenylthio | H | ethylthio |

Table 1 (Continued)

| A | $R_1$ | $R_2$ | $R_3$ | $R_4$ | $R_5$ |
|---|---|---|---|---|---|
| 2-amino-2-(4-hydroxyphenyl)acetyl | ethyl | ethyl | 4-hydroxymethylphenyl-sulfinyl | H | isopropylthio |
| 2-amino-2-(3-ethylsulfonylamino-phenyl)acetyl | cyclopropyl | | 4-acetylamino-3-methoxy-phenylsulfonyl | H | n-butylthio |
| 2-amino-2-(4-methylphenyl)acetyl | ethyl | n-butyl | methylsulfonyloxy | H | isobutylthio |
| 2-amino-2-(benzothien-3-yl)acetyl | ethyl | n-propyl | trifluoromethylsulfonyloxy | H | formamido |
| 2-hydroxy-2-(1,3,4-thiadiazol-2-yl)-acetyl | ethyl | Hydrogen | ethoxycarbonyl | H | Hydrogen |
| 2-carboxy-2-(benzothien-2-yl)acetyl | Hydrogen | ethyl | butoxycarbonyl | H | methoxy |
| 2-oxo-2-phenylacetyl | Hydrogen | methyl | 2-phenylethoxycarbonyl | H | ethoxy |
| 2-oxo-2-(2-aminothiazol-4-yl)acetyl | Hydrogen | n-butyl | 3-phenyl-(n-propoxy)carbonyl | H | propoxy |
| 2-(2-thienyl)-2-ethoxyiminoacetyl | methyl | ethyl | hydroxy | H | butoxy |
| 2-(2-chlorothiazol-4-yl)-2-methoxy-iminoacetyl | methyl | methyl | ethoxy | H | methylthio |
| 2-(2-aminothiazol-4-yl)-2-(2-carbamoyl-prop-2-yl)oxyiminoacetyl | methyl | n-butyl | propoxy | H | ethylthio |

<u>Table 1</u> (Continued)

| A | $R_1$ | $R_2$ | $R_3$ | $R_4$ | $R_5$ |
|---|---|---|---|---|---|
| acetyl | ethyl | methyl | heptanoyl | H | isopropylthio |
| n-propionyl | methyl | n-propyl | phenylmethoxy | H | n-butylthio |
| t-butyryl | cyclopropyl | | 2-phenylethoxy | H | isobutylthio |
| n-valeryl | n-propyl | n-butyl | 4-phenylheptanoyl | H | formamido |

A preferred group of (1-alkylsubstituted) and (1,1-dialkylsubstituted)carbacephalosporins is represented by the Formula I wherein R is the substituted methyl group

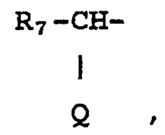

particularly those compounds wherein Q is amino and $R_7$ is phenyl, hydroxyphenyl, thienyl, or benzothienyl. Examples of such substituents, together with the CO moiety to which they are attached, are D-phenylglycyl, D-4-hydroxyphenylglycyl, D-2-thienylglycyl, D-benzothien-3-ylglycyl, and like functionalities.

A further preferred group is represented by Formula I wherein R is the group

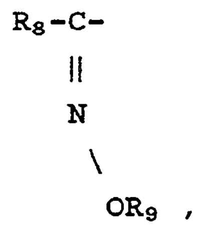

in the syn form.

Particularly preferred compounds are represented when $R_9$ is $C_1$-$C_4$ alkyl or a carboxy substituted alkyl group such as carboxymethyl, 2-carboxyethyl, 3-carboxypropyl, and 2-carboxy-2-propyl ; and $R_8$ is a five or six membered heterocyclic ring $R_8$ group, in particular, an amino substituted heterocyclic. Especially preferred heterocyclics are the 2-aminothiazole or 2-aminooxazole ring. Examples of such preferred RCO- groups are (2-aminothiazol-4-yl)(methoxyimino)acetyl, (2-aminooxazol-4-yl)(methoxyimino)acetyl, and the like.

A particularly preferred group is represented by Formula I wherein R is the group

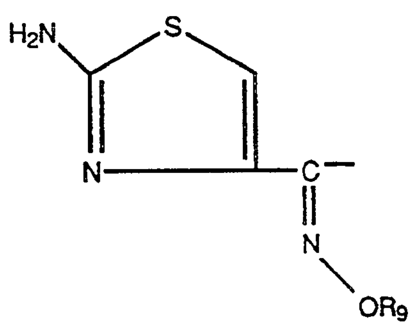

A further preferred group of (1,1-dialkylsubstituted) carbacephalosporins is represented by the Formula I wherein $R_1$ and $R_2$ are independently $C_1$-$C_2$ unbranched alkyl.

A further preferred group of (1-alkylsubstituted) carbacephalosporins is represented by the Formula I wherein $R_1$ or $R_2$ is hydrogen while the other is $C_1$-$C_2$ unbranched alkyl.

Particularly preferred compounds are represented when $R_1$ and $R_2$ are methyl, or cyclopropyl.

A further preferred group of (1-alkylsubstituted) and (1,1-dialkylsubstituted) carbacephalosporins is represented by the Formula I wherein $R_3$ is hydrogen, halo, methyl, vinyl, methoxy, methylthio, cyano, methylsulfonyl, or methylsulfonyloxy, trifluoromethylsulfonyl or trifluoromethylsulfonyloxy.

Particularly preferred compounds are represented when $R_3$ is chloro, methyl, or methoxy.

A further preferred group of (1-alkylsubstituted) and (1,1-dialkylsubstituted) carbacephalosporins is represented by the Formula I wherein $R_4$ is hydrogen.

A further preferred group of (1-alkylsubstituted) and (1,1-dialkylsubstituted) carbacephalosporins is represented by the Formula I wherein $R_5$ is hydrogen, methoxy, methylthio or formamido.

Particularly preferred compounds are represented when $R_5$ is hydrogen.

The preferred compounds represented by Formula I are those which are formed by choosing substituents

for A, $R_1$, $R_2$, $R_3$, $R_4$, and $R_5$ from the preferred groups represented above.

The compounds represented by Formula I can be prepared according to the route outlined in Scheme I. In Scheme I, A, $R_1$, $R_2$, $R_3$, $R_4$, and $R_5$ have the same meanings as defined above for Formula I.

The starting material (1) of Scheme I, when $R_1$ is $C_1$-$C_4$ unbranched alkyl and $R_2$ is hydrogen or $C_1$-$C_4$ unbranched alkyl, is prepared either by reacting a di-substituted aldehyde with allyl alcohol, according to the known procedure of Salomon and Ghosh, Org. Syn., 62, 125 (1984) or by reacting a propenyl ether with allyl alcohol, according to the known procedure of Montgomery et al., J. Am. Chem. Soc., 89, 923 (1967). The starting material (1) of Scheme I, when $R_1$ and $R_2$ form cyclopropyl, is prepared by photodecarbonylation taught by Morton et al., J. Am. Chem. Soc., 92, 4349 (1970).

The imine (2) of Scheme I is formed by reacting the substituted 4-pentenal (1) with benzylamine in an unreactive aromatic solvent under dehydrating conditions. Water can be removed by refluxing with a Dean-Stark apparatus or through the use of a dehydrating agent, such as anhydrous magnesium sulfate. The reaction can be carried out at temperatures that range from 0-100°C in solvents such as benzene or toluene.

The β-lactam (4) of Scheme I is prepared according to the general procedure of Evans and Sjogren, Tetrahedron Letters, 26, 3783 (1985), who reacted a chiral oxazolidone acetyl chloride (3) with an imine (2) in a [2 + 2] cycloaddition.

The chiral auxilary and benzyl group are removed under known deprotection conditions taught in EP 209,352 to provide (5).

The resultant amine (5) of Scheme I, can then be acylated with any specific side-chain at the 7- position by known cephalosporin or carbacephalosporin chemistry.

The carboxylic acid (7) of Scheme I, can be produced from olefin (6) under a variety of olefin oxidation conditions, such as, potassium permanganate in acetone-water-acetic acid, potassium permanganate and 18-crown-6 in benzene, or ozone/hydrogen peroxide in methylene chloride-methanol.

The acid (7) of Scheme I, is converted to the corresponding β-keto ester (8) by the method taught by Brooks et al., Angew. Chem. Int. Ed., 18, 72 (1979), using carbonyl diimidazole in tetrahydrofuran at 0-100°C for 1-10 hours and then Masamune reagent is added to the mixture and stirred for another 12-24 hours.

The substituted carbacepham derivative (10) of Scheme I and II, is prepared by conversion of (8) to the diazo derivative (9) by reacting (8) with p-carboxybenzenesulfonyl azide, Hunig's base, and acetonitrile for 12-24 hours. This is followed by a rhodiumcatalyzed diazo insertion reaction as taught by Christensen et al., Tetrahedron Letters, 21, 31 (1980), to provide the 3-hydroxy compound (10).

The 3-enol (10) can be isolated as the mesylate ester or triflate ester. US 4,673,737 teaches the preparation of 3-triflate from the 3-hydroxy (10) by O-acylation. These esters can be used as intermediates for preparing other $R_3$ substituted compounds defined above in Formula 1.

The carbacephem compounds where $R_3$ is halo are prepared according to US 4,673,737 where the 3-triflate intermediate is reacted with a lithium halide.

According to US 4,855,418, compounds where $R_3$ is $C_1$-$C_6$ alkyl, $C_1$-$C_6$ alkyl substituted by cyano, carboxy, halogen, or amino, phenyl or substituted phenyl can be prepared by reacting the 3-triflate intermediate in an inert solvent in the presence of an alkali metal halide with a tin tranfer reagent.

The 3-cyano substituted compounds can be prepared according to EP 154,253 in which the 3-mesylate intermediate is reacted with an alkali metal derivative of cyanide such as sodium cyanide or potassium cyanide.

The 3-thio, sulfinyl, and sulfonyl substituted compounds are prepared according to US 4885291, in which the 3-triflate intermediate is reacted with a sulfinate salt, $R_3SO_2^-A^+$, where $A^+$ represents an alkali metal cation such as lithium, sodium or potassium.

The 3-ester compounds are prepared according to EP 299,728 in which the 3-triflate intermediate is reacted in an inert solvent in the presence of palladium (0) with carbon monoxide and an alcohol represented by the formula $R_{13}OH$.

The 3-ether compounds are prepared according to known methods taught in Spry, et al., Heterocycles, 24, 1653, (1986), by way of a Mitsunobu reaction in which the 3-hydroxy intermediate is reacted with trivalent phosphorous and an azodicarboxylate.

The 7α-substituted-1-carbacephalosporins (11) represented by formula 1 are prepared by known chemistry. The 7α-alkoxy substituted compounds are prepared according to US 3,994,885 ; the 7α-alkylthio are prepared according to Gordon and Sykes, Cephamycin Antibiotic, Ch. 3, p. 280 of Chemistry and Biology of β-lactam Antibiotics, Vol. 1, edited by Morin and Gorman, 1982 ; and the 7α-formamido substituted compounds are prepared according to US 4,539,159.

SCHEME I

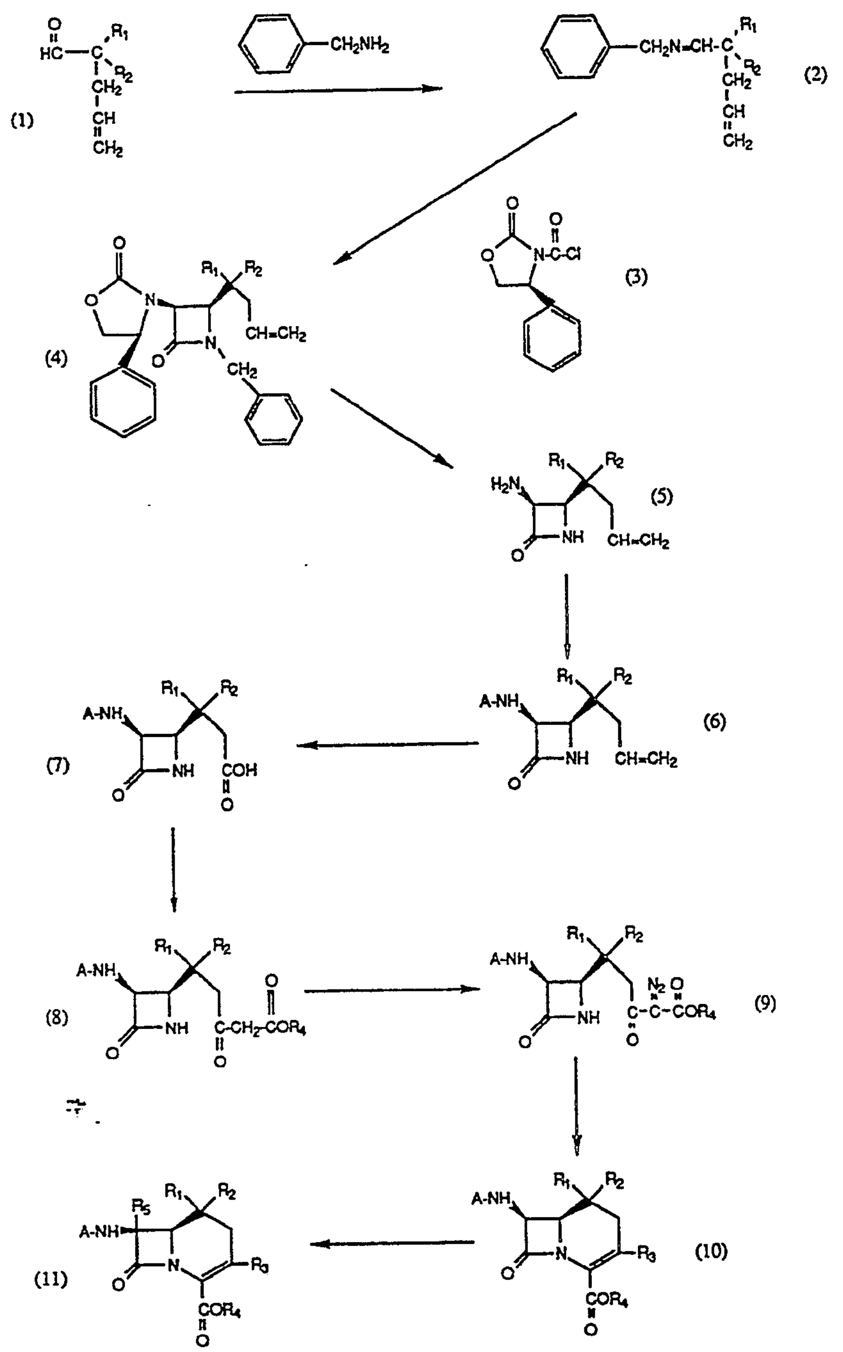

(1) (2) (3) (4) (5) (6) (7) (8) (9) (10) (11)

The following Examples are provided to further illustrate the invention. It is not intended that the invention be limited in scope by reason of any of the following Examples.

In the following Examples, the terms nuclear magnetic resonance spectra, mass spectra, infra-red spectra, ultraviolet spectra, elemental analysis and high performance liquid chromatography are abbreviated NMR, MS, IR, UV, Anal. and HPLC, respectively. In addition, the adsorption maxima listed for the IR spectra are only those

of interest and not all of the maxima observed.

In the Examples the following abbreviations have the indicated meanings : DMF = dimethylformamide ; THF = tetrahydrofuran ; DIPEA = diisopropyethylamine ; and t-Boc = t-butyloxy carbonyl.

In conjunction with the NMR spectra, the following abbreviations are used : "s" is singlet, "d" is doublet, "dd" is doublet of doublets, "br. s" is broad singlet, "br. d" is a broad doublet, "t" is triplet, "q" is quartet, "m" is multiplet and "dm" is a doublet of multiplets. "J" indicates the coupling constant in Hertz. "DMSO/$d_6$" is dimethyl sulfoxide where all protons have been replaced with deuterium.

The NMR spectra were obtained on a Varian Associates EM-390 90 MHz or T-60 60 MHz instrument, on a Jeol fX-90 Q90 MHz instrument or on a QE-300 MHz instrument. The chemical shifts are expressed in δ values (parts per million downfield from tetramethylsilane). The field desorption mass spectra were taken on a Varian-MAT 731 Spectrometer using carbon dendrite emitters. Mass spectral data were obtained on a CEC 21-110 instrument or a Varian MAT-731 spectrometer.

Example #1

5,5-Dimethyl-3-[(methylsulfonyl)oxy]-8-oxo-7-[(phenoxyacetyl)-amino]-1-azabicyclo[4.2.0]oct-2-ene-2-carboxylic acid

A. 2,2-Dimethyl-4-pentenal

2,2-Dimethyl-4-pentenal was prepared according to the procedure of Salomon and Ghosh, Org. Syn., 62, 125 (1984). A solution of 216 g (3.0 mol) of isobutyraldehyde, 116 g (2.0 mol) of allyl alcohol, 0.8 g (.004 mol) of p-toluenesulfonic acid and 460 ml of p-cymene was heated at reflux while any water produced was collected with a Dean-Stark trap. The resulting mixture was purified by distillation with a fractionating column of glass helices. The fraction collected between 120°C and 150°C was redistilled under the same conditions and three fractions collected between 90°C and 135°C to provide 100 g of a clear liquid containing about 90% pure 2,2-dimethyl-4-pentenal.

$^1$H NMR (90 MHz, $CDCl_3$) δ 9.4 (s,1H), 6.0-5.4 (m, 1H), 5.2-4.9 (m, 2H), 2.2 (d, 2H, J = 7.0Hz), and 1.05 (s, 6H).

B. N-[(2,2-Dimethyl)-4-pentenylidene]benzenemethanamine

A suspension of 2.8 g (20.0 mmol) of 90% 2,2-dimethyl-4-pentenal, 2.0 ml (18.3 mmol) of benzylamine and 25 ml of benzene was stirred at reflux with a Dean-Stark trap for three hours. The mixture containing N-[(2,2-dimethyl)-4-pentenylidene]benzenemethanamine was used directly in the reaction labeled D below.

C. 2-Oxo-4-phenyl-3-oxazolidoneacetyl chloride

To a solution of 4.42 g (20.0 mmol) of 2-oxo-4-phenyl-3-oxazolidineacetic acid and 2.62 ml (30.0 mmol) of oxalyl chloride in 50 ml of methylene chloride was added about ten drops of DMF. Following completion of gas evolution (about 2.5 hours), the mixture was concentrated under vacuum with toluene. The residue containing 2-oxo-4-phenyl-3-oxazolidineacetyl chloride was used directly in the following reaction labeled D.

D. 3-[2-(1,1-Dimethyl-3-butenyl)-4-oxo-1-(phenylmethyl)-3-azetidinyl]-4-phenyl-2-oxazolidinone

The residue containing 2-oxo-4-phenyl-3-oxazolidinoneacetyl chloride prepared as described in step C above was combined with 50 ml of methylene chloride. The resulting solution was cooled to about-78°C with an external dry ice/acetone bath and 4.2 ml of triethylamine was slowly added. The resulting mixture was stirred at-78°C for 15 minutes and the solution of N-[(2,2-dimethyl)-4-pentenylidene]benzenemethanamine in benzene prepared as described above in step B was slowly added to the mixture dropwise. The resulting mixture was stirred at-78°C for 30 minutes, at 0°C for four hours and finally at room temperature overnight. The mixture was diluted with ethyl acetate and washed in order with water, an aqueous saturated sodium bicarbonate solution, water, a solution of 1N hydrochloric acid, water and an aqueous saturated sodium chloride solution. The resulting organic phase was dried over anhydrous magnesium sulfate, filtered and concentrated under vacuum to provide 6.5 g of a yellow oil. Column chromatography on silica gel provided 5.0 g of 3-[2-(1,1-dimethyl-3-butenyl)-4-oxo-1-(phenylmethyl)-3-azetidinyl]-4-phenyl-2-oxazolidinone as a white foam.

$^1$H NMR (90 MHz, $CDCl_3$) δ 7.6-7.2 (m, 10H), 6.0-5.4 (m, 1H), 5.2-4.5 (m, 5H), 4.3-4.1 (m, 3H), 3.4 (d, 1H, J = 5Hz), 2.1 (d, 2H, J = 7Hz), and 1.0 (s, 6H).

IR ($CHCl_3$) 1774, 1753, 1419, 1402, 1390, 1380, and 1125 $cm^{-1}$.

OR : $[a]_D$ =+103.6 (c = 0.5 in MeOH).

MS : m/e 404 (m+)

Anal : Calculated for $C_{25}H_{28}N_2O_3$

Theoretical : C, 74.23 ; H, 6.98 ; N, 6.93.

Found : C, 74.49 ; H, 7.13 ; N, 6.78.

E. cis-3-Amino-4-[1,1-dimethyl-3-butenyl]-2-azetidinone

To a solution of 664 mg (94.8 mmol) of lithium in 250 ml of ammonia cooled to about-78°C was added a solution of 3.83 g (9.48 mmol) of 3-[2-(1,1-dimethyl-3-butenyl)-4-oxo-1-(phenylmethyl)-3-azetidinyl]-4-phenyl-2-oxazolidinone and 2.68 ml (28.44 mmol) of t-butanol in 50 ml of THF. The mixture was stirred at about-78°C for one hour and quenched with 1,2-dichloroethane. The mixture was concentrated under vacuum overnight. The residual white powder was dissolved in aqueous 1N HCl, and the pH of the solution was raised to 11 with sodium hydroxide. The mixture was extracted with chloroform/isobutanol. The organic extracts were dried over anhydrous magnesium sulfate, filtered and concentrated under vacuum to provide 1.5 g of cis-3-amino-4-(1,1-dimethyl-3-butenyl)-2-azetidinone as a white solid, used in the next step without purification.

F. cis-3-[(Phenoxyacetyl)amino]-4-(1,1-dimethyl-3-butenyl)-2-azetidinone

A suspension of 1.5 g (8.9 mmol) of cis-3-amino-4-(1,1-dimethyl-3-butenyl)-2-azetidinone, 3.19 g of sodium bicarbonate, 1.31 ml of phenoxyacetyl chloride in 25 ml of water and 25 ml of acetonitrile was stirred at room temperature for 6 hours. The mixture was diluted with a saturated sodium bicarbonate solution and extracted with methylene chloride. The organic extracts were combined, dried over anhydrous magnesium sulfate, filtered and concentrated under vacuum to afford 2.6 g of cis-3-[(phenoxyacetyl)amino]-4-(1,1-dimethyl-3-butenyl)-2-azetidinone as a white solid.

$^1H$ NMR (300 MHz, DMSO-$d_6$) δ 8.82 (d, 1H, J = 7Hz), 8.38 (s, 1H), 7.4-6.8 (m, 5H), 6.82-6.6 (m, 1H), 5.15 (dd, 1H, J = 5, 7Hz), 5.1-4.9 (m, 2H), 4.6 (ABq, 2H), 3.5 (d, 1H, J = 5Hz), 1.9 (d, 2H, J = 7Hz), 0.92 (s, 3H), and 0.89 (s, 3H)

IR ($CDCl_3$) : 3420, 1775, 1690, 1530, 1480, and 1230 $cm^{-1}$.

MS : m/e = 302 (m+)

UV : (EtOH) λ max 214 (E = 13,854), 269 (E = 1587), and 276 (E = 1280).

OR : $[\alpha]_D$ =+62.5 (C = 0.5 in MeOH).

Anal : Calculated for $C_{17}H_{22}N_2O_3$

Theoretical : C, 67.53 ; H, 7.33 ; N, 9.26.

Found : C, 67.65 ; H, 7.29 ; N, 9.54.

G. cis-4-Oxo-3-[(phenoxyacetyl)amino]-2-azetidine-(B,B-dimethyl)propanoic acid

A solution of 2.0 g (6.62 mmol) of cis-3-[(phenoxyacetyl)amino]-4-(1,1-dimethyl-3-butenyl)-2-azetidinone, 4.18 g (26.48 mmol) of potassium permanganate and 6 ml of acetic acid in 100 ml of acetone and 100 ml of water was stirred at about 0°C for three hours. The mixture was stored in a freezer overnight, and quenched with sodium sulfite. To the mixture was added ethyl acetate and 1N hydrochloric acid, and the mixture was saturated with sodium chloride. The mixture was extracted with ethyl acetate. The organic extracts were combined, dried over anhydrous magnesium sulfate, filtered and concentrated <u>in vacuo</u> to provide 2.45 g of cis-4-oxo-3-[(phenoxyacetyl)amino]-2-azetidine-(3,3-dimethyl)-propanoic acid as a white solid.

$^1H$ NMR (300 MHz, DMSO-$d_6$) δ 12.1 (br. s, 1H), 8.85 (d, 1H, J = 8Hz), 8.4 (s, 1H), 7.4-6.8 (m, 5H), 5.15 (dd, 1H, J = 8, 5Hz), 4.6 (ABq, 2H), 3.6 (d, 1H, J = 5 Hz), 2.1 (s, 2H), 0.95 (s, 6H)

IR ($CDCl_3$) : 3500-2900 (br), 1775, 1710, 1690, 1600, 1525, and 1250 $cm^{-1}$.

MS : m/e = 321 (m + H)

Anal : Calculated for $C_{16}H_{20}N_2O_5$ :

Theoretical : C, 59.99 ; H, 6.29 ; N, 8.74.

Found : C, 59.69 ; H, 6.19 ; N, 8.13.

H. cis-B,4-Dioxo-3-[(phenoxyacetyl)amino]-2-azetidine-(D,D-dimethyl)pentanoic acid (4-nitrophenyl)methyl ester

Carbonyldiimidazole (2.58 g, 0.016 mol) was added to a solution of 1.7214 g (5.37 mmol) of cis 4-oxo-3-[(phenoxyacetyl)amino]-2-azetidine-(3,3-dimethyl)-propanoic acid in 150 ml of THF at 0°C. The mixture was stirred at 0°C for 5 hours and 5.16 g of Masamune reagent was added. The mixture was stirred at 0°C for 8 hours, and then at room temperature for 10 hours. The THF was removed <u>in vacuo</u> and the resulting residue was dissolved in ethyl acetate. The organic solution was washed with 1N HCl, water, an aqueous saturated sodium bicarbonate solution, and an aqueous saturated sodium chloride solution. The organic solution was dried over anhydrous magnesium sulfate, filtered, and concentrated under vacuum to provide the desired compound. This compound was further purified by column chromatography over silica gel while eluting with ethyl acetate : toluene (3 : 7, v : v) to provide 583 mg of cis-B,4-dioxo-3-[(phenoxyacetyl)amino]-2-azetidine-(D,D-dimethyl)pentanoic acid (4-nitrophenyl)methyl ester as a white solid.

$^1H$ NMR (300 MHz, DMSO-$d_6$) δ 8.95 (d, 1H J = 8Hz), 8.38 (s, 1H), 8.22 (d, 2H, J = 8Hz), 7.65 (d, 2H, J = 8Hz), 7.3-6.9 (m, 5H), 5.28 (ABq, 2H), 5.1 (dd, 1H, J = 8, 5Hz), 4.6 (s, 2H), 3.6 (s, 2H), 3.5 (d, 1H, J = 5Hz) 2.5 (ABq, 2H), and 0.95 (s, 6H).

IR ($CDCl_3$) : 3420, 3020, 1770, 1719, 1688, 1609, 1601, 1525, 1496, 1442, 1317, 1290, and 1260 $cm^{-1}$

MS : m/e 498 (m+)
Anal : Calculated for $C_{25}H_{27}N_3O_8$
Theoretical : C, 60.36 ; H, 5.47 ; N, 8.45.
Found : C, 60.43 ; H, 5.61 ; N, 8.63.

I. cis-A-Diazo-B,4-dioxo-3-[(phenoxyacetyl)amino]-2-azetidine (D,D-dimethyl)pentanoic acid (4-nitrophenyl)-methyl ester

A solution of 517.9 mg (1.042 mmol) of cis-B,4-dioxo-3-[(phenoxyacetyl)amino]-2-azetidine (D,D-dimethyl)pentanoic acid (4-nitrophenyl)methyl ester, 1.6 ml of DIPEA and 528 mg of p-carboxybenzenesulfonyl azide in 50 ml of acetonitrile was stirred at room temperature for 18 hours. The solvent was removed <u>in vacuo</u> and the residue was combined with ethyl acetate and a saturated sodium bicarbonate solution. The organic layer was separated and dried over anhydrous magnesium sulfate, filtered, and concentrated under vacuum to provide 713 mg of a yellow solid. The solid was purified with column chromatography over silica gel while eluting with ethyl acetate : toluene (3 : 7, v : v) to provide 410.5 mg of cis-A-diazo-B,4-dioxo-3-[(phenoxyacetyl)amino]-2-azetidine-(D,D-dimethyl)pentanoic acid (4-nitrophenyl)methyl ester as a white solid.

$^1H$ NMR (300 MHz, DMSO-$d_6$) δ 8.78 (d, 1H, J = 8Hz), 8.38 (s, 1H), 8.25 (d, 2H, J = 8Hz), 7.65 (d, 2H, J = 8Hz), 7.3-6.9 (m, 5H), 5.35 (s, 2H), 5.15 (dd, 1H, J = 8, 5Hz), 4.58 (ABq, 2H), 3.65 (d, 1H, J = 5Hz), 2.75 (ABq, 2H), 0.98 (s, 3H), and 0.95 (s, 3H).
IR ($CDCl_3$) 3400, 2990, 2144, 1772, 1716, 1700, 1560, 1500, 1350, and 1238 $cm^{-1}$.
MS : m/e 524 (m+)
Anal : Calculated for $C_{25}H_{25}N_5O_8$
Theoretical : C, 57.36 ; H, 4.81 ; N, 13.38.
Found : C, 57.14 ; H, 4.96 ; N, 13.14.

J. 5,5-Dimethyl-3-[(methylsulfonyl)oxy]-8-oxo-7-[(phenoxyacetyl)amino]-1-azabicyclo[4.2.0]oct-2-ene-2-carboxylic acid (4-nitrophenyl)methyl ester

A solution of 363.4 mg (0.6948 mmol) of cis-A-diazo-B,4-dioxo-3-[(phenoxyacetyl)amino]-2-azetidine (D,D-dimethyl)pentanoic acid (4-nitrophenyl)methyl ester and 10 mg of rhodium acetate in 70 ml of chloroform was refluxed for three hours, and then cooled to 0°C. To the solution was added 0.362 ml of DIPEA (2.08 mmol) and 0.06 ml methanesulfonyl chloride (0.7643 mmol) and the mixture was stirred at 0°C for 16 hours. The mixture was washed with an aqueous saturated sodium bicarbonate solution, dried over magnesium sulfate, filtered and concentrated under vacuum to provide the desired compound. The compound was further purified by column chromatography over silica gel while eluting with toluene/ethyl acetate to provide 209 mg of 5,5-dimethyl-3-[(methylsulfonyl)oxy]-8-oxo-7-[(phenoxyacetyl)amino]-1-azabicyclo[4.2.0]oct-2-ene-2-carboxylic acid (4-nitrophenyl)methyl ester as a white foam.

$^1H$ NMR (300 MHz, DMSO-$d_6$) δ 8.95 (d, 1H, J = 8Hz), 8.25 (d, 2H, J = 8Hz), 7.72 (d, 2H, J = 8Hz), 7.3-6.9 (m, 5H), 5.5 (dd, 1H, J = 5, 8Hz), 5.4 (ABq, 2H), 4.62 (s, 2H), 3.7 (d, 1H, J = 5Hz), 3.4 (s, 3H), 2.45 (ABq, 2H), 0.98 (s, 3H), and 0.92 (s, 3H).
IR ($CDCl_3$) 3400, 3010, 1777, 1737, 1695, 1610, 1525, 1495, and 1293 $cm^{-1}$.
MS : m/e = 574 (m+)
Anal : Calculated for $C_{26}H_{27}N_3O_{10}S$
Theoretical : C, 54.44 ; H, 4.74 ; N, 6.33.
Found : C, 56.61 ; H, 5.52 ; N, 6.35.

K. 5,5-Dimethyl-3-[(methylsulfonyl)oxy]-8-oxo-7-[phenoxyacetyl)amino]-1-azabicyclo[4.2.0]oct-2-ene-2-carboxylic acid

Excess zinc dust was added to a solution of 5,5-dimethyl-3-[(methylsulfonyl)oxy]-8-oxo-7-[(phenoxyacetyl)amino]-1-azabicyclo[4.2.0]oct-2-ene-2-carboxylic acid (4-nitrophenyl)methyl ester in 7 ml of DMF and 5 ml of 1N HCl and stirred for two hours at room temperature. The mixture was diluted with ethyl acetate, washed five times with 1N HCl and dried over anhydrous magnesium sulfate. The mixture was filtered and concentrated under vacuum to provide 40.1 mg of a yellow solid. The solid was further purified by acid base workup to provide 5,5-dimethyl-3-[(methylsulfonyl) oxy]-8-oxo-7-[(phenoxyacetyl)amino]-1-azabic yclo[4.2.0]oct-2-ene-2-carboxylic acid.

$^1H$ NMR (300 MHz, DMSO-$d_6$) δ 13.6 (br. s, 1H), 8.92 (d, 1H, J = 8Hz), 7.3-6.9 (m, 5H), 5.42 (dd, 1H, J = 5, 8Hz), 4.63 (s, 2H), 3.62 (d, 1H, J = 5Hz), 3.38 (s, 3H), 2.38 (ABq, 2H), 0.98 (s, 3H), and 0.92 (s, 3H).
IR (KBr) : 3500-2900 (br), 1777, 1730, 1689, 1363, and 1210 $cm^{-1}$.
MS : m/e = 438 (m+)

<u>Example #2</u>

5,5-Dimethyl-3-chloro-8-oxo-7-(D-α-phenylgmlcylamino)-1-azabicyclo[4.2.0]oct-2-ene-2-carboxylic acid, trifluoroacetate salt

A. 5,5-Dimethyl-3-hydroxy-8-oxo-7-[(phenoxyacetyl) amino]-1-aza-bicyclo[4.2.0]oct-2-ene-2-carboxylic acid (4-nitro-phenyl)methyl ester

A solution of 524.0 mg (1.0 mmol) of cis-A-diazo-B,4-dioxo-3-[(phenoxyacetyl)amino]-2-azetidine (D,D-dimethyl)pentanoic acid (4-nitrophenyl)methyl ester and 10 mg of rhodium acetate in 100 ml of chloroform is refluxed for three hours. The mixture is concentrated to afford the desired compound, which is purified by chromatography.

B. 5,5-Dimethyl-3-chloro-8-oxo-7-amino-1-azabicyclo [4.2.0]oct-2-ene-2-carboxylic acid (4-nitrophenyl-)methyl ester hydrochloride

5,5-Dimethyl-3-hydroxy-8-oxo-7-[(phenoxyacetyl)-amino]-1-azabicyclo[4.2.0]oct-2-ene-2-carboxylic acid (4-nitrophenyl)methyl ester is treated with triphenylphosphite dichloride according to the general procedure of Hatfield et al. in "Recent Advances in the Chemistry of β-Lactam Antibiotics" ; Gregory, G.I., Ed. ; The Royal Society of Chemistry : Burlington House, London, 1980 ; p 109, to afford the title compound.

C. 5,5-Dimethyl-3-chloro-8-oxo-7-(N-BOC-D-α-phenylglycylamino)-1-azabicyclo[4.2.0]oct-2-ene-2-carboxylic acid (4-nitrophenyl)methyl ester

Phosphorous oxychloride (1.0 mmol) is added to a solution of t-BOC-D-α-phenylglycine (1.0 mmol), pyridine (1.5 mmol) and 5,5-dimethyl-3-chloro-8-oxo-7-amino-1-azabicyclo[4.2.0]oct-2-ene-2-carboxylic acid (4-nitrophenyl)methyl ester in 25 ml of methylene chloride at 0°C. The mixture is stirred at 0°C for 3 hours, then diluted with ethyl acetate. The organic layer is washed with 1N HCl, water, saturated bicarbonate, and brine, dried over magnesium sulfate, filtered and concentrated to afford the desired compound.

D. 5,5-Dimethyl-3-(chloro)-8-oxo-7-(N-BOC-D-α-phenylglycylamino)-1-azabicyclo[4.2.0]oct-2-ene-2-carboxylic acid

Zinc dust (100.0 mmol) is added to a solution of 5,5-dimethyl-3-(chloro)-8-oxo-7-(N-BOC-D-α-phenylglycyl-amino)-1-azabicyclo[4.2.0]oct-2-ene-2-carboxylic acid (4-nitrophenyl)methyl ester (1.0 mmol) in 60 ml of a 1/1/1 solution of THF-DMF-1N HCl at room temperature. The mixture is stirred for 2 hours, then diluted with ethyl acetate. The desired product is obtained from an aqueous saturated sodium bicarbonate solution and a 1N hydrochloric acid workup.

E. 5,5-Dimethyl-3-(chloro)-8-oxo-7-(D-α-phenylgly-cylamino)-1-azabicyclo[4.2.0]oct-2-ene-2-carboxylic acid, trifluoroacetate salt

A solution of 5,5-dimethyl-3-(chloro)-8-oxo-7-(D-α-phenylglycyl-amino)-1-azabicyclo[4.2.0]oct-2-ene-2-carboxylic acid in neat trifluoroacetic acid is stirred at room temperature for 4 hours. The title compound is obtained by solvent removal and trituration with diethyl ether.

Example #3

5,5-Dimethyl-3-[(trifluoromethylsulfonyl)oxy]-8-oxo-7-[(phenoxyacetyl)amino]-1-azabicyclo[4.2.0]oct-2-ene-2-carboxylic acid

A. 5,5-Dimethyl-3-[(trifluoromethylsulfonyl)oxy]-8-oxo-7-[(phenoxyacetyl)amino]-1-azabicyclo[4.2.0]-oct-2-ene-2-carboxylic acid (4-nitrophenyl)methyl ester

A solution of 524.0 mg (1.0 mmol) of cis-A-diazo-B,4-dioxo-3-[(phenoxyacetyl)amino]-2-azetidine (D,D-dimethyl)pentanoic acid (4-nitrophenyl)methyl ester and 10 mg of rhodium acetate in 100 ml of chloroform is refluxed for three hours then is cooled to 0°C. To this solution is added 3.62 ml of DIPEA (2.08 mmol) and 0.17 ml trifluoromethanesulfonic anhydride (1.0 mmol) and the resulting mixture is stirred at 0°C for 16 hours. The mixture is quenched with aqueous saturated sodium bicarbonate solution. The separated organic layer is dried over magnesium sulfate, filtered, and concentrated to provide the desired compound which is purified by chromatography.

B. 5,5-Dimethyl-3-[(trifluoromethylsulfonyl)oxy]-8-oxo-7-[(phenoxyacetyl)amino]-1-azabicyclo[4.2.0]oct-2-ene-2-carboxylic acid

5,5-Dimethyl-3-[(trifluoromethylsulfonyl)oxy]-8-oxo-7-[(phenoxyacetyl)amino]-1-azabicyclo[4.2.0]oct-2-ene-2-carboxylic acid (4-nitrophenyl)methyl ester is treated according to the procedure described in Example 1, part K to afford the desired compound.

Example #4

5,5-Dimethyl-3-chloro-8-oxo-7-[2-(2-aminothiazol-4-yl)-2-methoximinoacetylamino]1-azabicyclo[4.2.0]-oct-2-ene-2-carboxylic acid

A. 5,5-Dimethyl-3-chloro-8-oxo-7-amino-1-azabicyclo[4.2.0]oct-2-ene-2-carboxylic acid

A mixture of 1.5 ml of concentrated HCl and 0.5 ml of water is added to a solution of 5,5-dimethyl-3-chloro-8-oxo-7-amino-1-azabicyclo[4.2.0]oct-2-ene-2-carboxylic acid (4-nitrophenyl)methyl ester hydrochloride (1.0 mmol) in 3 ml of DMF at 0°C. Zinc dust (200 mg) is slowly added in small portions and the resulting mixture is stirred at 0°C for 4 hours. The pH is adjusted to approximately 3.5 with solid sodium bicarbonate and the resulting precipitated product is collected by filtration.

B. 5,5-Dimethyl-3-chloro-8-oxo-7-[2-(2-aminothiazole-4-yl)-2-methoximinoacetylamino]-1-azabicyclo[4.2.0]oct-2-ene-2-carboxylic acid

To a solution of 5,5-dimethyl-3-chloro-8-oxo-7-amino-1-azabicyclo[4.2.0]oct-2-ene-2-carboxylic acid (1.0 mmol) in 5 ml of acetone and 5 ml of water, adjusted to pH 8 with sodium bicarbonate, is added the hydroxybenzotriazole active ester of syn-(2-amino-4-thiazolyl)methoximinoacetic acid (1.0 mmol). The mixture is stirred at pH 8 for 4 hours. The mixture is diluted with water and washed with methylene chloride. The aqueous layer is acidified to pH 3 and extracted with methylene chloride. The organic extracts are dried over magnesium sulfate, filtered, and concentrated to afford the desired compound.

Example #5

5,5-Dimethyl-3-chloro-8-oxo-7-{[2(R)-phenyl-(4-ethyl-2,3-dioxopiperazin-1-ylcarbonyl)amino]acetylamino}-1-azabicyclo[4.2.0]oct-2-ene-2-carboxylic acid

A mixture of 4-ethyl-2,3-dioxopiperazin-1-yl-carbonyl chloride (1.0 mmol), 5,5-dimethyl-3-(chloro)-8-oxo-7-[amino]-1-azabicyclo[4.2.0]oct-2-ene-2-carboxylic acid (1.0 mmol), 5 ml of propylene oxide, and 2.5 ml of bis(trimethylsilyl)acetamide in 15 ml of acetonitrile is stirred for 1 hour at 0°C. The mixture is concentrated, and then triturated with diethyl ether to afford the desired compound.

Example #6

5-α-Methyl-3-(chloro)-8-oxo-7-(D-α-phenylglycylamino)-1-azabicyclo[4.2.0]oct-2-ene-2-carboxylic acid, triflouroacetate salt and 5-β-Methyl-3-chloro-8-oxo-7-(D-α-phenylglycylamino)-1-azabicyclo[4.2.0] oct-2-ene-2-carboxylic acid, trifluoroacetate salt

A. 3-[2-(1-α-Methyl-3-butenyl)-4-oxo-1-(phenyl-methyl)-3-azetidinyl]-4-phenyl-2-oxazolidinone and 3-[2-(1-β-methyl-3-butenyl)-4-oxo-1-(phenylmethyl)-3-azetidinyl]-4-phenyl-2-oxazolidinone

2-Methyl-4-pentenal (prepared according to the procedure of Montgomery et al., J. Am. Chem. Soc., 89, 923 (1967), is substituted for 2,2-dimethyl-4-pentenal, and is reacted as described in Example 1, parts A., B., C., and D., to afford the desired compounds which are separated by chromatography.

B. 5-α-Methyl-3-(chloro)-8-oxo-7-(D-α-phenylglycylamino)-1-azabicyclo[4.2.0]oct-2-ene-2-carboxylic acid, trifluoroacetate salt and 5-β-Methyl-3-(chloro)-8-oxo-7-(D-α-phenylglycylamino)-1-azabicyclo[4.2.0] oct-2-ene-2-carboxylic acid, trifluoroacetate salt

3-[2-(1-α-Methyl-3-butenyl)-4-oxo-1-(phenyl-methyl)-3-azetidinyl]-4-phenyl-2-oxazolidinone and 3-[2-(1-ß-methyl-3-butenyl)-4-oxo-1-(phenylmethyl)-3-azetidinyl]-4-phenyl-2-oxazolidinone are reacted as described in Example 1, parts E., F., G., H., and I., and Example 2, parts A., B., C., D., and E., to afford the title compounds.

Example #7

5-Spirocyclopropyl-3-chloro-8-oxo-7-(D-α-phenyl-glycylamino)-1-azabicyclo[4.2.0]oct-2-ene-2-carboxylic acid, trifluoracetate salt

A. 3-[2-(1-Spirocyclopropyl-3-butenyl)-4-oxo-1-(phenylmethyl)-3-azetidinyl]-4-phenyl-2-oxazolidinone

1-Allylcyclopropanecarboxyaldehyde (Morton et al., J. Am. Chem. Soc., 92, 4349 (1970)) is substituted for 2,2-dimethyl-4-pentenal, and is reacted as described in Example 1, parts A., B., C., and D., to afford the desired compound which is purified by chromatography.

B. 5-Spirocyclopropyl-3-chloro-8-oxo-7-(D-α-phenylglycylamino)-1-azabicyclo[4.2.0]oct-2-ene-2-carboxylic acid, trifluoracetate salt

3-[2-(1-Spirocyclopropyl-3-butenyl)-4-oxo-1-(phenylmethyl)-3-azetidinyl]-4-phenyl-2-oxazolidinone is reacted as described in Example 1, parts E., F., G., H., and I., and Example 2, parts A., B., C., D., and E., to afford the title compound.

The compounds of Formula I inhibit the growth of certain pathogenic organisms as demonstrated by standard agar-plate disc-diffusion tests. Table I summarizes the results of such tests with the compound of Example 1 listed above. Antimicrobial activity is measured by the size (diameter in mm) of the observed zone in which growth of the microorganism is inhibited by the test compound.

Table 2

Zone of Bacterial and Fungal Growth Inhibition[1]
By Agar-Plate Disc-Diffusion Test

| Organism | Dosage 1 mg/ml | 10 mg/ml |
|---|---|---|
| Staphylococcus aureas X1 | 24 | 17 |
| Bacillus subtilis X12 | 15 | 10 |
| Bacillus subtilis X12M[2] | 35 | 15 |
| Sarcina lutea X186 | 37 | 20 |
| Bacillus stearothermophilus C451 | 22 | 24 |
| Mycobacterium avium X85 | -[3] | - |
| Escherichia coli X161 | 10 | 12 |
| Escherichia coli X161M[2] | tr[4] | 15 |
| Pseudomonas solanacearum X185 | 21 | tr |
| Escherichia coli X580 | 29 | 32 |
| Saccharomyces pastorianus X52 | - | - |
| Neurospora crassa 846 | - | - |
| X657 | - | - |
| X142 | tr | tr |
| X45 | - | - |

*Numerals and letters following the names of test microorganisms refer to the strains.

1) The test compounds were dissolved in water at a concentration of 1.0 mg/ml ; a 7 mm disc was dipped into the suspension and then placed on the agar surface ; cultures were incubated 24-48 hours at 25-35°C.
2) Growth on minimal nutrient agar
3) The symbol "-" indicates no observable zone
4) The symbol "tr" indicates a trace zone

The antimicrobial compounds of this invention are useful for the therapeutic or prophylactic treatment of infections in warm-blooded animals caused by both gram-positive, gram-negative and acid-fast bacteria.

The antimicrobial can be administered orally, parenterally (e.g. intravenously, intramuscularly or subcutaneously) or as a topical ointment or solution in treating bacterial infections of warm-blooded animals.

A further aspect of this invention is the pharmaceutical compositions of the antimicrobial compounds of Formula I. In particular, these pharmaceutical compositions are useful for the control of gram-positive and gram-negative bacterial infections and comprise a suitable vehicle and a therapeutically effective amount of the antimicrobial compounds of Formula I.

With regard to compositions for oral administration (e.g. tablets and capsules), the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose and starch ; fillers and carriers, for example corn starch, gelatin, lactose, sucrose, microcrystalline cellulose, kaolin, mannitol, dicalcium phosphate, sodium chloride and alginic acid ; disintegrators such as microcrystalline cellulose, corn starch, sodium starch glycolate, alginic acid ; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups ; or b) a dry powder to be reconstituted with water or another suitable vehicle before use. When used in conjunction with such oral liquid preparations, the term "suitable vehicle" means conventional additives such as suspending agents, for example, sorbitol, syrup, methyl cellulose, glucose/sugar syrup, gelatin, hydroxyethylcellulose, carboxymethylcellulose, aluminum stearate gel or hydrogenated edible oils, for example almond oil, fractionated coconut oil, oily esters, propylene glycol or ethyl alcohol ; and preservatives such as methyl or propyl p-hydroxybenzoates or sorbic acid.

The pharmaceutical composition can also be for intravenous (IV) use. Specifically, a water soluble form of the antimicrobial compound can be dissolved in one of the commonly used intravenous fluids and administered by infusion. When used in conjunction with compositions for IV use, the term "suitable vehicle" means such fluids as physiological saline, Ringer's solution or 5% dextrose solution.

For intramuscular preparations a sterile formulation of a suitable salt form of the antimicrobial compound (for example, the hydrochloride salt or sodium salt) can be formulated with a "suitable vehicle". Examples of such sterile formulations are a suitable salt form either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

Topical compositions can be formulated with "suitable vehicles" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with "suitable vehicles" such as long- or quick-release bases.

The antimicrobial compounds of Formula I can be used as surface disinfectants. Solutions containing as little as 0.1 percent by weight of the antimicrobial compound are effective for disinfecting purposes. Preferably, such solutions also can contain a detergent or other cleansing agent. The solutions are useful for disinfecting objects such as glassware, dental and surgical instruments, and surfaces such as walls, floors, and tables in areas where maintenance of sterile conditions is important, for example, hospitals, food-preparation areas, and the like.

The antimicrobial compounds of Formula I can also be formulated in unit dosage form in sterile vials, sterile plastic pouches containing a port with a septum, or sterile, hermetically sealed ampoules. The antimicrobial compound (or the corresponding pharmaceutically-acceptable salt) may be a dry powder or in crystalline or lyophilized form. The amount of the antimicrobial compound per unit dosage may vary from about 250 milligrams to about 10 grams.

A "therapeutically effective amount" of the antimicrobial compounds of Formula I is from approximately 3.5 mg to about 50 mg of compound per kilogram of body weight. This amount generally totals from about 1 gram to about 27 grams per day for an adult human.

A further aspect of this invention is a method for treating or controlling infectious diseases caused by gram-positive and gram-negative organisms in warm blooded animals. This method comprises administering to the animal a therapeutically effective amount of the instant antimicrobial compounds. A typical daily dose for an adult human in this method is from about 1 gram to about 12 grams.

In practicing this method, the antibiotic can be administered in a single daily dose or in multiple doses per day. The treatment regime may require administration over extended periods of time, e.g., for several days or for from two to three weeks. The amount administered per dose or the total amount administered will depend on such factors as the nature and severity of the infection, the age and general health of the patient, the tolerance of both the patient and the microorganism or microorganisms involved in the infection to the antimicrobial compound.

The following formulation examples represent specific pharmaceutical formulations employing compounds comprehended by the present method. The formulations may employ as active compounds any of the compounds of Formula I or a pharmaceutically acceptable salt or biologically labile ester thereof. The examples are illustrative only and are not intended to limit the scope of the invention in any way.

Formulation 1

Hard gelatin capsules are prepared using the following ingredients :

| | Quantity (mg/capsule) |
|---|---|
| Example 1 | 250 |
| Starch dried | 200 |
| Magnesium stearate | 10 |

The above ingredients are mixed and filled into hard gelatin capsules in 460 mg quantities.

Formulation 2

A tablet formula is prepared using the ingredients below :

| | Quantity (mg/tablet) |
|---|---|
| Example 2 | 250 |
| Cellulose, microcrystalline | 400 |
| Silicon dioxide, fumed | 10 |
| Stearic acid | 5 |
| Magnesium stearate | 10 |

The components are blended and compressed to form tablets each weighing 675 mg.

Formulation 3

An aerosol solution is prepared containing the following components :

| | Weight |
|---|---|
| Example 3 | 0.25 |
| Ethanol | 29.75 |
| Propellant 22 (Chlorodifluoromethane) | 70.00 |

The active compound is mixed with ethanol and the mixture added to a portion of the propellant 22, cooled to-30°C and transferred to a filling device. The required amount is then placed in a stainless steel container and diluted with the remainder of the propellant. The valve units are then fitted to the container.

Formulation 4

Tablets each containing 60 mg of active ingredient are made up as follows :

| | |
|---|---|
| Example 4 | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |

The difluoronucleoside, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules each containing 80 mg of medicament are made as follows :

| | |
|---|---|
| Example 5 | 80 mg |
| Starch | 59 mg |
| Microcrystalline cellulose | 59 mg |
| Silicone fluid | 2 mg |

The active ingredient, cellulose, starch and magnesium stearate are blended, passed through a No. 45 mesh U.S. sieve, and filled into hard gelatin capsules in 200 mg quantities.

Formulation 6

Suppositories each containing 225 mg of medicament are made as follows :

| | |
|---|---|
| Example 6 | 225 mg |
| Saturated fatty acid glycerides to | 2 mg |

The nucleoside is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

As intravenous formulation is prepared as follows :

| | |
|---|---|
| Example 7 | 100 mg |
| Isotonic saline | 1000 ml |

The solution of the above ingredients is administered intravenously at a rate of 1 ml/minute to a mammal in need of treatment.

## Claims

1. A compound of the formula

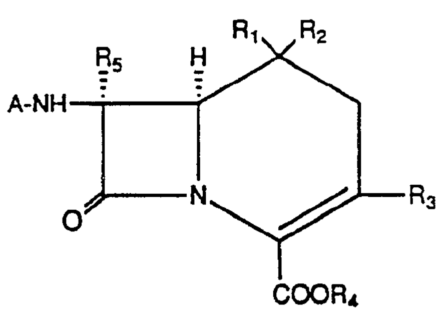

I

wherein A is hydrogen, an amino-protecting group, or an acyl group

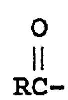

wherein R is hydrogen, $C_1$-$C_6$ alkyl, $C_1$-$C_6$ alkyl substituted by cyano, carboxy, halogen, amino, $C_1$-$C_4$ alkoxy, $C_1$-$C_4$ alkylthio, trifluoromethyl, or trifluoromethylthio, naphthyl, an optionally substituted phenyl group represented by the formula

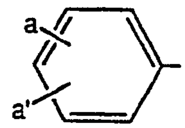

wherein a and a' independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, $C_1$-$C_4$ alkanoylamino, $C_1$-$C_4$ alkylsulfonylamino, carboxy, carbamoyl, hydroxymethyl, aminomethyl, or carboxymethyl ;
a group represented by the formula

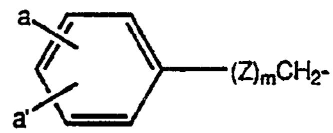

wherein Z is O or S, and m is 0 or 1 ;
a heteroarylmethyl group represented by the formula

$R_6$-$CH_2$-

wherein $R_6$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino ;
a substituted methyl group represented by the formula

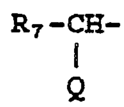

wherein $R_7$ is cyclohex-1,4-dienyl, or an optionally substituted phenyl group represented by the formula

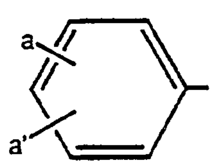

wherein a and a' have the above defined meanings, or $R_7$ is $R_6$ as defined above, and Q is hydroxy, $C_1$-$C_4$ alkanoyloxy, carboxy, sulfo, amino ;
or R is a keto group or an oximino-substituted group represented by the formulae

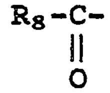

or

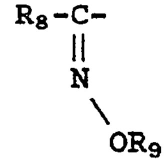

wherein $R_8$ is $R_6$ or $R_7$ as defined above and $R_8$ is hydrogen, $C_1$-$C_4$ alkyl, or a carboxysubstituted alkyl or cycloalkyl group represented by the formula

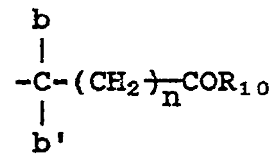

wherein b and b′ independently are hydrogen or $C_1$-$C_3$ alkyl, or b and b′, when taken together with the carbon to which they are bonded, form a 3- to 6-membered carbocyclic ring, n is 0-3, and $R_{10}$ is hydroxy, $C_1$-$C_4$ alkoxy, amino, $C_1$-$C_4$ alkylamino, or di($C_1$-$C_4$ alkyl)amino ;
$R_1$ and $R_2$ independently are hydrogen or $C_1$-$C_4$ unbranched alkyl, but $R_1$ and $R_2$ are not both hydrogen, or $R_1$ and $R_2$, together, form cyclopropyl ;
$R_3$ is hydrogen, halo, $C_1$-$C_6$ alkyl, $C_1$-$C_6$ alkyl substituted by cyano, carboxy, halogen or amino, phenyl, substituted phenyl, cyano ; a group of the formula

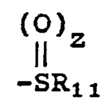

wherein z is 0, 1 or 2 and $R_{11}$ is $C_1$-$C_6$ alkyl, $C_1$-$C_6$ alkyl substituted by cyano, carboxy, halogen, or amino, phenyl, substituted phenyl, a group of the formula

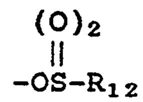

wherein $R_{12}$ is methyl or trifluoromethyl ; a group of the formula

$-COOR_{13}$

wherein $R_{13}$ is $C_1$-$C_6$ alkyl or $C_7$-$C_{12}$ arylalkyl ; a group of the formula

$-OR_{14}$

wherein $R_{14}$ is hydrogen, $C_1$-$C_6$ alkyl, or $C_7$-$C_{12}$ arylalkyl ;
$R_4$ is hydrogen, a biologically labile group, or a carboxy-protecting group ;
$R_5$ is hydrogen, $C_1$-$C_4$ alkoxy, $C_1$-$C_4$ alkylthio, or a formamido group ;
or a pharmaceutically acceptable salt thereof.

2. A compound of Claim 1 wherein $R_1$ and $R_2$ independently are $C_1$-$C_4$ unbranched alkyl.

3. A compound of Claim 2 wherein $R_1$ and $R_2$ are methyl.

4. A compound of any one of Claims 1, 2 or 3 wherein $R_4$ is hydrogen, or a pharmaceutically acceptable salt or biologically labile ester thereof.

5. A compound of either of Claim 1 or 4 wherein $R_3$ is halo, hydroxy, $C_1$-$C_6$ alkyl or $C_1$-$C_6$ alkylsulfonyloxy.

6. A compound of Claim 5 wherein $R_3$ is halo.

7. A compound of either of Claims 1 or 5 wherein A is hydrogen, phenylglycyl, or 2-aminooxazol-4-yl(methoxyimino)acetyl.

8. A pharmaceutical formulation comprising an antibiotically effective amount of a compound of formula I as claimed in any one of Claims 1 to 6 wherein $R_4$ is hydrogen, or a pharmaceutically acceptable salt or biologically labile ester thereof, associated with one or more pharmaceutically acceptable carriers, excipients or diluents therefor.

9. An antibiotic compound of Claim 1 wherein $R_4$ is hydrogen or a pharmaceutically acceptable salt or biologically labile ester thereof, for use as a pharmaceutical.

10. A process for preparing a compound of formula I, as defined in claim 1, wherein $R_4$ is hydrogen, or a pharmaceutically acceptable salt or a biologically labile ester thereof, comprising deprotecting a compound of formula I, as defined in claim 1, wherein $R_4$ is a carboxy-protecting group, and optionally adding a biologically labile $R_4$ group or salifying the product wherein $R_4$ is hydrogen.

## Claims for the following Contracting State : GR

1. A process for preparing a compound of the formula

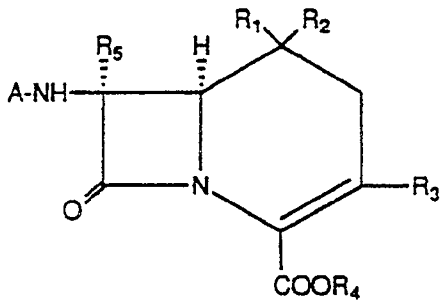

I

wherein A is an acyl group

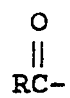

wherein R is hydrogen, $C_1$-$C_6$ alkyl, $C_1$-$C_6$ alkyl substituted by cyano, carboxy, halogen, amino, $C_1$-$C_4$ alkoxy, $C_1$-$C_4$ alkylthio, trifluoromethyl, or trifluoromethylthio, naphthyl, an optionally substituted phenyl group represented by the formula

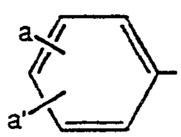

wherein a and a' independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, $C_1$-$C_4$ alkanoylamino, $C_1$-$C_4$ alkylsulfonylamino, carboxy, carbamoyl, hydroxymethyl, aminomethyl, or carboxymethyl ;
a group represented by the formula

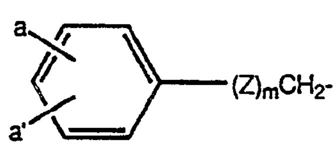

wherein Z is O or S, and m is 0 or 1 ;
a heteroarylmethyl group represented by the formula

$R_6$-$CH_2$-

wherein $R_6$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino ;
a substituted methyl group represented by the formula

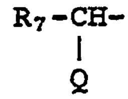

wherein $R_7$ is cyclohex-1,4-dienyl, or an optionally substituted phenyl group represented by the formula

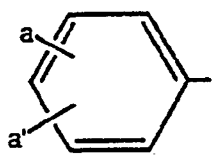

wherein a and a' have the above defined meanings, or $R_7$ is $R_6$ as defined above, and Q is hydroxy, $C_1$-$C_4$ alkanoyloxy, carboxy, sulfo, amino ;
or R is a keto group or an oximino-substituted group represented by the formulae

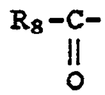 or 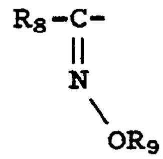

wherein $R_8$ is $R_6$ or $R_7$ as defined above and $R_9$ is hydrogen, $C_1$-$C_4$ alkyl, or a carboxysubstituted alkyl or cycloalkyl group represented by the formula

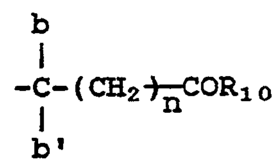

wherein b and b' independently are hydrogen or $C_1$-$C_3$ alkyl, or b and b', when taken together with the carbon to which they are bonded, form a 3- to 6-membered carbocyclic ring, n is 0-3, and $R_{10}$ is hydroxy, $C_1$-$C_4$ alkoxy, amino, $C_1$-$C_4$ alkylamino, or di($C_1$-$C_4$ alkyl)amino ;
$R_1$ and $R_2$ independently are hydrogen or $C_1$-$C_4$ unbranched alkyl, but $R_1$ and $R_2$ are not both hydrogen, or $R_1$ and $R_2$, together, form cyclopropyl ;
$R_3$ is hydrogen, halo, $C_1$-$C_6$ alkyl, $C_1$-$C_6$ alkyl substituted by cyano, carboxy, halogen or amino, phenyl, substituted phenyl, cyano ; a group of the formula

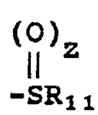

wherein z is 0, 1 or 2 and $R_{11}$ is $C_1$-$C_6$ alkyl, $C_1$-$C_6$ alkyl substituted by cyano, carboxy, halogen, or amino, phenyl, substituted phenyl, a group of the formula

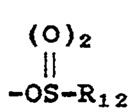

wherein $R_{12}$ is methyl or trifluoromethyl ; a group of the formula

$-COOR_{13}$

wherein $R_{13}$ is $C_1$-$C_6$ alkyl or $C_7$-$C_{12}$ arylalkyl ; a group of the formula

$-OR_{14}$

wherein $R_{14}$ is hydrogen, $C_1$-$C_6$ alkyl, or $C_7$-$C_{12}$ arylalkyl ;
$R_4$ is hydrogen or a biologically labile group ;
$R_5$ is hydrogen, $C_1$-$C_4$ alkoxy, $C_1$-$C_4$ alkylthio, or a formamido group ;
or a pharmaceutically acceptable salt thereof, comprising deprotecting a compound of formula I wherein $R_4$ is a carboxy-protecting group, and optionally adding a biologically labile group or salifying the product wherein $R_4$ is hydrogen.

**2.** A process of Claim 1 for preparing a compound wherein $R_1$ and $R_2$ independently are $C_1$-$C_4$ unbranched alkyl.

**3.** A process of Claim 2 for preparing a compound wherein $R_1$ and $R_2$ are methyl.

**4.** A process of any one of Claims 1 to 3 for preparing a compound wherein $R_4$ is hydrogen, or a pharmaceutically acceptable salt thereof.

**5.** A process of either of Claims 1 or 4 for preparing a compound wherein $R_3$ is halo, hydroxy, $C_1$-$C_6$ alkyl or $C_1$-$C_6$ alkylsulfonyloxy.

**6.** A process of Claim 5 for preparing a compound wherein $R_3$ is halo.

**7.** A process of either of Claims 1 or 5 for preparing a compound wherein A is hydrogen, phenylglycyl, or 2-aminooxazol-4-yl(methoxyimino)acetyl.

**8.** A process for preparing a pharmaceutical formulation comprising admixing a compound of formula I, as defined in any one of Claims 1 to 7, with one or more pharmaceutically-acceptable carriers, diluents or excipients therefor.

**9.** A compound of the formula

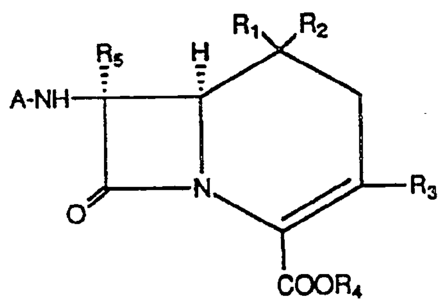

I

wherein A is hydrogen, an amino-protecting group, or an acyl group

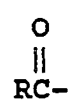

wherein R is hydrogen, $C_1$-$C_6$ alkyl, $C_1$-$C_6$ alkyl substituted by cyano, carboxy, halogen, amino, $C_1$-$C_4$ alkoxy, $C_1$-$C_4$ alkylthio, trifluoromethyl, or trifluoromethylthio, naphthyl, an optionally substituted phenyl group represented by the formula

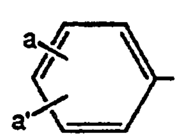

wherein a and a' independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, $C_1$-$C_4$ alkanoylamino, $C_1$-$C_4$ alkylsulfonylamino, carboxy, carbamoyl, hydroxymethyl, aminomethyl, or carboxymethyl ;
a group represented by the formula

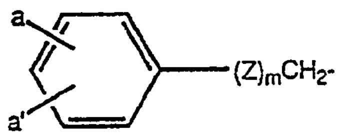

wherein Z is O or S, and m is 0 or 1 ;
a heteroarylmethyl group represented by the formula

$$R_6\text{-}CH_2\text{-}$$

wherein $R_6$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino ;
a substituted methyl group represented by the formula

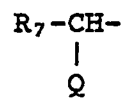

wherein $R_7$ is cyclohex-1,4-dienyl, or an optionally substituted phenyl group represented by the formula

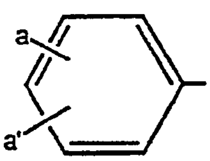

wherein a and a' have the above defined meanings, or $R_7$ is $R_6$ as defined above, and Q is hydroxy, $C_1$-$C_4$ alkanoyloxy, carboxy, sulfo, amino ;
or R is a keto group or an oximino-substituted group represented by the formulae

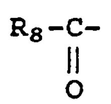

or

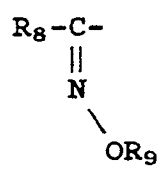

wherein $R_8$ is $R_6$ or $R_7$ as defined above and $R_9$ is hydrogen, $C_1$-$C_4$ alkyl, or a carboxysubstituted alkyl or cycloalkyl group represented by the formula

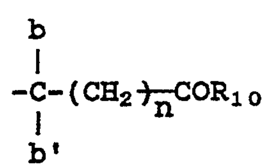

wherein b and b' independently are hydrogen or $C_1$-$C_3$ alkyl, or b and b', when taken together with the carbon to which they are bonded, form a 3- to 6-membered carbocyclic ring, n is 0-3, and $R_{10}$ is hydroxy, $C_1$-$C_4$ alkoxy, amino, $C_1$-$C_4$ alkylamino, or di($C_1$-$C_4$ alkyl)amino ;
$R_1$ and $R_2$ independently are hydrogen or $C_1$-$C_4$ unbranched alkyl, but $R_1$ and $R_2$ are not both hydrogen,

or $R_1$ and $R_2$, together, form cyclopropyl ;
$R_3$ is hydrogen, halo, $C_1$-$C_6$ alkyl, $C_1$-$C_6$ alkyl substituted by cyano, carboxy, halogen or amino, phenyl, substituted phenyl, cyano ; a group of the formula

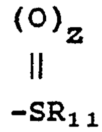

wherein z is 0, 1 or 2 and $R_{11}$ is $C_1$-$C_6$ alkyl, $C_1$-$C_6$ alkyl substituted by cyano, carboxy, halogen, or amino, phenyl, substituted phenyl, a group of the formula

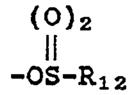

wherein $R_{12}$ is methyl or trifluoromethyl ; a group of the formula

$-COOR_{13}$

wherein $R_{13}$ is $C_1$-$C_6$ alkyl or $C_7$-$C_{12}$ arylalkyl ; a group of the formula

$-OR_{14}$

wherein $R_{14}$ is hydrogen, $C_1$-$C_6$ alkyl, or $C_7$-$C_{12}$ arylalkyl ;
$R_4$ is a carboxy-protecting group ;
$R_5$ is hydrogen, $C_1$-$C_4$ alkoxy, $C_1$-$C_4$ alkylthio, or a formamido group.

**Claims for the following Contracting State : ES**

1. A process for preparing a compound of the formula

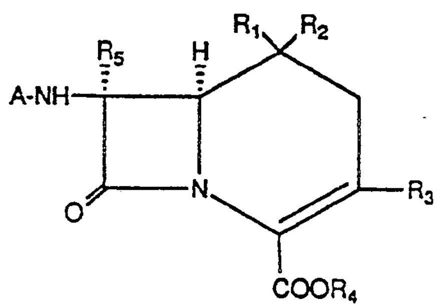

I

wherein A is an acyl group

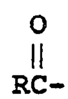

wherein R is hydrogen, $C_1$-$C_6$ alkyl, $C_1$-$C_6$ alkyl substituted by cyano, carboxy, halogen, amino, $C_1$-$C_4$ alkoxy, $C_1$-$C_4$ alkylthio, trifluoromethyl, or trifluoromethylthio, naphthyl, an optionally substituted phenyl group represented by the formula

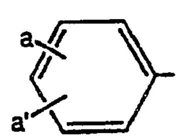

wherein a and a' independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, $C_1$-$C_4$ alkanoylamino, $C_1$-$C_4$ alkylsulfonylamino, carboxy, carbamoyl, hydroxymethyl, aminomethyl, or carboxymethyl ;
a group represented by the formula

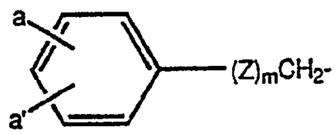

wherein Z is O or S, and m is 0 or 1 ;
a heteroarylmethyl group represented by the formula

$$R_6-CH_2-$$

wherein $R_6$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino ;
a substituted methyl group represented by the formula

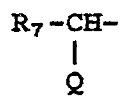

wherein $R_7$ is cyclohex-1,4-dienyl, or an optionally substituted phenyl group represented by the formula

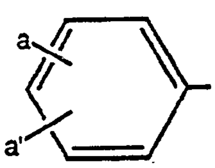

wherein a and a' have the above defined meanings, or $R_7$ is $R_6$ as defined above, and Q is hydroxy, $C_1$-$C_4$ alkanoyloxy, carboxy, sulfo, amino ;
or R is a keto group or an oximino-substituted group represented by the formulae

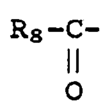

or

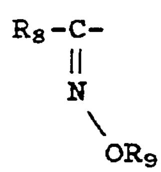

wherein $R_8$ is $R_6$ or $R_7$ as defined above and $R_9$ is hydrogen, $C_1$-$C_4$ alkyl, or a carboxysubstituted alkyl or cycloalkyl group represented by the formula

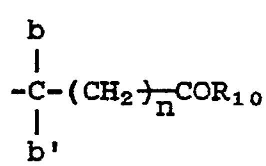

wherein b and b' independently are hydrogen or $C_1$-$C_3$ alkyl, or b and b', when taken together with the carbon to which they are bonded, form a 3- to 6-membered carbocyclic ring, n is 0-3, and $R_{10}$ is hydroxy, $C_1$-$C_4$ alkoxy, amino, $C_1$-$C_4$ alkylamino, or di($C_1$-$C_4$ alkyl)amino ;

$R_1$ and $R_2$ independently are hydrogen or $C_1$-$C_4$ unbranched alkyl, but $R_1$ and $R_2$ are not both hydrogen, or $R_1$ and $R_2$, together, form cyclopropyl ;
$R_3$ is hydrogen, halo, $C_1$-$C_6$ alkyl, $C_1$-$C_6$ alkyl substituted by cyano, carboxy, halogen or amino, phenyl, substituted phenyl, cyano ; a group of the formula

$$\overset{(O)_z}{\overset{||}{-SR_{11}}}$$

wherein z is 0, 1 or 2 and $R_{11}$ is $C_1$-$C_6$ alkyl, $C_1$-$C_6$ alkyl substituted by cyano, carboxy, halogen, or amino, phenyl, substituted phenyl, a group of the formula

$$\overset{(O)_2}{\overset{||}{-OS-R_{12}}}$$

wherein $R_{12}$ is methyl or trifluoromethyl ; a group of the formula

$$-COOR_{13}$$

wherein $R_{13}$ is $C_1$-$C_6$ alkyl or $C_7$-$C_{12}$ arylalkyl ;
a group of the formula

$$-OR_{14}$$

wherein $R_{14}$ is hydrogen, $C_1$-$C_6$ alkyl, or $C_7$-$C_{12}$ arylalkyl ;
$R_4$ is hydrogen or a biologically labile group ;
$R_5$ is hydrogen, $C_1$-$C_4$ alkoxy, $C_1$-$C_4$ alkylthio, or a formamido group ;
or a pharmaceutically acceptable salt thereof, comprising deprotecting a compound of formula I wherein $R_4$ is a carboxy-protecting group, and optionally adding a biologically labile group or salifying the product wherein $R_4$ is hydrogen.

2. A process of Claim 1 for preparing a compound wherein $R_1$ and $R_2$ independently are $C_1$-$C_4$ unbranched alkyl.

3. A process of Claim 2 for preparing a compound wherein $R_1$ and $R_2$ are methyl.

4. A process of any one of Claims 1 to 3 for preparing a compound wherein $R_4$ is hydrogen, or a pharmaceutically acceptable salt thereof.

5. A process of either of Claims 1 or 4 for preparing a compound wherein $R_3$ is halo, hydroxy, $C_1$-$C_6$ alkyl or $C_1$-$C_6$ alkylsulfonyloxy.

6. A process of Claim 5 for preparing a compound wherein $R_3$ is halo.

7. A process of either of Claims 1 or 5 for preparing a compound wherein A is hydrogen, phenylglycyl, or 2-aminooxazol-4-yl(methoxyimino)acetyl.

8. A process for preparing a pharmaceutical formulation comprising admixing a compound of formula I, as defined in any one of Claims 1 to 7, with one or more pharmaceutically-acceptable carriers, diluents or excipients therefor.